# EUROPEAN PATENT APPLICATION

(11) **EP 2 322 519 A1**
(43) Date of publication of application: **18.05.2011**
(21) Application number: 09173959.9
(22) Date of filing: 23.10.2009
(51) Int. Cl.: C07D 333/56

(54) **A process for preparing benzo[b]thiophene derivatives**

(71) Applicant: Hexal AG, 83607 Holzkirchen (DE)
(72) Inventor: Schickaneder, Christian, 01309 Dresden (DE); Delling, Axel, 01445 Radebeul (DE)
(74) Representative: Prüfer & Partner GbR European Patent Attorneys

(57) **Abstract**

The present invention relates in general to the field of organic chemistry, and in particular to the preparation of benzo[b]thiophene derivatives. These benzo[b]thiophene derivatives are useful as intermediates in the synthesis of pharmaceutically active agents such as raloxifene or derivatives thereof.

## Description

### Field of the invention

The present invention relates in general to the field of organic chemistry, and in particular to the preparation of benzo[b]thiophene derivatives. These benzo[b]thiophene derivatives can be used as intermediates in the synthesis of pharmaceutically active agents such as raloxifene or derivatives thereof.

### Background of the invention

Compounds comprising a benzo[b]thiophene moiety are important intermediates for the preparation of active pharmaceutical active agents. For example, raloxifene (6-hydroxy-2-(4-hydroxyphenyl)-3-[4-(2-piperidinoethoxybenzoyl]-benzo[b]thiophene) is a selective estrogen receptor modulator possessing estrogen agonist-like actions on bone tissues and serum lipids, while displaying efficient estrogen antagonist properties in breast and uterus.

The object of the present invention is to provide an improved process for preparing benzo[b]thiophene derivatives representing valuable key intermediates for the preparation of pharmaceutically active agents such as raloxifene or derivatives thereof.

### Summary of the invention

Various aspects, advantageous features and preferred embodiments of the present invention as summarized in the following items, respectively alone or in combination, contribute to solving the object of the invention:
(1) A process for preparing a crystalline hydrate of a compound of formula XI 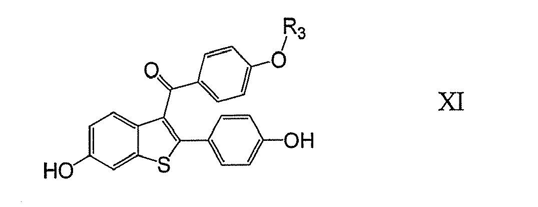 wherein R₃ is 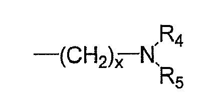 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring, preferably a 5- to 7-membered ring, more preferably a 5- or 6-membered ring, in particular a 6-membered ring,
wherein said process comprises the steps of:
a) providing a free base of the compound of formula XI, a salt of the compound of formula XI or a solvate form of a salt of the compound of formula XI comprising at least one solvated organic solvent,
b) dissolving said compound provided in step a) with a mixture of an antisolvent for a free base of a compound of formula XI and an aqueous solution of a proton acceptor in an at least stoichiometric molar amount or higher relative to the compound of formula XI provided in step a),
c) adjusting the pH of the mixture resulting from step b) to 7-9 by adding a proton donator,
d) optionally seeding the mixture resulting from step c) in order to induce crystallisation,
e) allowing hydrate of compound of formula XI to precipitate, preferably to crystallize, optionally under agitation, and
f) separating the crystalline hydrate of compound of formula XI obtained in step e).
The term "alkyl" as used herein means straight, branched or cyclic hydrocarbons of 1 to 12 carbon atoms, preferably 1 to 8 carbon atoms and more preferably 1 to 6 carbon atoms.
The term "aryl" as used herein means hydrocarbon aryls, preferably single or condensed six-membered rings, more preferably phenyl or naphthyl, in particular phenyl.
The term "alkylaryl" as used herein means that the aforementioned aryl moieties are incorporated into the aforementioned straight or branched alkyl moieties either at one of the proximal or distal ends of the alkyl chain or between the aforementioned alkyl chains. For example, proximal end means for R₁ e.g. adjacent to the oxygen bound to the benzene ring of the benzo[b]thiophene moiety of compound of formula VI, while distal means the terminal carbon of the alkyl moiety which is furthermost from said oxygen.
The term "substituted" as employed herein means that the aforementioned alkyl, aryl and alkylaryl groups have one, two or three linear or branched C₁-C₆ alkyl substituents.
The term "free base of a compound of formula XI" as employed herein means a compound of formula XI wherein the nitrogen of R₃ is in its free basic form, that is, said nitrogen is not protonated and thus distinct from the nitrogen atom from R₃ of a salt of a compound of formula XI.
The term "solvate form" as used herein means a crystalline solid adduct wherein a stoichiometric or nonstoichiometric amount of at least one organic solvent is incorporated in said crystalline solid. In a particular embodiment, a first solvent and a second solvent is incorporated in said crystalline solid.
The term "proton acceptor" as used herein means a Brønsted base which provides for accepting the proton of a salt of a compound of formula XI or a solvate thereof in order to convert said salt of a compound of formula XI or a solvate thereof into a free base of a compound of formula XI. Furthermore, the kind and amount of proton acceptor used in step b) is selected with the proviso that compound of formula XI provided in step a) is dissolved under the conditions of step b).
The term "antisolvent for a free base of a compound of formula XI" as used herein means a solvent which provides for dissolution of the compound of formula XI provided in step a) under the conditions of step b), while said antisolvent for a free base of a compound of formula XI provides for precipitation/crystallization of a free base of a compound of formula XI in form of its hydrate under the conditions of steps c), d) and e) respectively.
The term "proton donator" as used herein means a Brønsted acid which provides for donating proton(s). In step c) of the present process, the amount of proton donator is selected such that a compound resulting from step b) is converted into a free base of a compound of formula XI.
The term "seeding" as used herein means any conditions known in the art may assist in the induction of crystallisation, such as adding a seed crystal, scratching the glass containing the mixture resulting from step c) in order to promote the formation of seed crystals and/or cooling the mixture resulting from step c) below ambient temperature. Preferably, seeding is effected by adding a seed crystal.
The term "hydrate of a compound of formula XI" specifies a crystalline solid adduct based on compound of formula XI in form of its free base, wherein a stoichiometric or nonstoichiometric amount of water is incorporated in said crystalline solid. The hydrate of compound of formula XI may be free of organic solvent, but optionally may additionally have an organic solvent incorporated. In a particular embodiment, said crystalline solid adduct comprises both water and an organic solvent being an antisolvent for a free base of a compound of formula XI.
The procedural concept according to this aspect of the invention provides for a hydrate of a compound of formula XI representing a highly valuable intermediate for the preparation of pharmaceutically active agents such as raloxifene or derivatives thereof, since the formation of the hydrate form of compound of formula XI provides for a highly effective purification step in the synthetic path for preparing a compound of formula XI. Furthermore, the present process for preparing a hydrate of a compound of formula XI is universally applicable to a free base of a compound of formula XI, a salt of a compound of formula XI and a solvate form of a salt of a compound of formula XI. The hydrate of the compound of formula XI obtained in step f) comprises compound of formula XI in form of its free base wherein the nitrogen atom of R₃ is not protonated. Thus, said hydrate furthermore represents a versatile compound for producing a pharmaceutically composition - due to its high purity, it may be used either directly or merely further converted into a pharmaceutically acceptable salt for the purpose of formulating a pharmaceutical composition.
(2) The process according to item (1), wherein the hydrate of a compound of formula XI comprises 2.8 to 4.7 % by weight of water relative to a hydrate of a compound of formula XI, preferably 3.2 to 4.2 % by weight, more preferably 3.4 to 3.9 % by weight.
(3) The process according to item (1) or (2), wherein said antisolvent for a free base of a compound of formula XI is acetone.
According to this embodiment of the invention, an antisolvent for a free base of a compound of formula XI is provided which is particularly suitable for providing precipitation or crystallisation.
(4) The process according to any one of items (1) to (3), wherein the hydrate of compound of formula XI comprises said antisolvent in an amount of up to 15 % by weight relative to a hydrate of a compound of formula XI, preferably up to 9.2 % by weight, more preferably up to 7.4 % by weight and in particular 0.1 to 4.9 % by weight.
(5) The process according to any one of items (1) to (4), wherein the obtained crystalline hydrate of a compound of formula XI is a hydrate of raloxifene with the formula 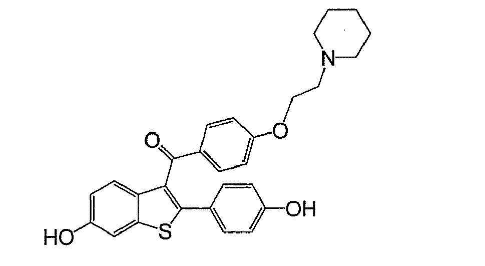 , preferably said hydrate of raloxifene exhibits an X-ray diffraction (XRD) pattern having at least signals at the following 2θ values (± 0.5° respectively):

| | | | | | | | | | | |
|---|---|---|---|---|---|---|---|---|---|---|
| **2theta[°]** | 8.13 | 13.13 | 15.18 | 18.11 | 18.39 | 18.65 | 23.07 | 23.49 | 23.76 | 26.02 |

, more preferably said hydrate of raloxifene exhibits an X-ray diffraction (XRD) pattern having at least signals at the following 2θ values (± 0.5° respectively):

| | | | | | | | | | | |
|---|---|---|---|---|---|---|---|---|---|---|
| **2theta[°]** | 8.13 | 9.54 | 12.46 | 13.13 | 13.93 | 15.18 | 16.15 | 16.9 | 17.9 | 18.11 |
| | 18.39 | 18.65 | 19.44 | 20.63 | 21.84 | 22.08 | 22.43 | 23.07 | 23.49 | 23.76 |
| | 24.63 | 24.95 | 26.02 | 26.32 | 26.49 | 28.08 | 31.25 | 31.69 | 35.33 | 40.36 |

In this preferred embodiment, 2θ values may be exact or substantially correspond with a tolerance of ±0.2° or even ±0.5° with the given 2θ values.
(6) The process according to any one of items (1) to (5), wherein the proton acceptor is selected from the group consisting of inorganic bases or organic amine bases, preferably alkaline metal hydroxides, earth alkaline metal hydroxides, ammonia and triethylamine, more preferably alkaline metal hydroxides, even more preferably sodium hydroxide and potassium hydroxide, and in particular sodium hydroxide.
(7) The process according to any one of items (1) to (6), wherein the at least stoichiometric amount of proton acceptor applied in step b) is 1 to 10 molar equivalents relative to the compound of formula XI provided in step a), preferably 1.5 to 8 molar equivalents, more preferably 3 to 7 molar equivalents and in particular 4.2 to 6 molar equivalents.
(8) The process according to any one of items (1) to (7), wherein the volume ratio of antisolvent for a free base of a compound of formula XI to aqueous proton acceptor of the mixture of step b) is in range from 1 to 2, preferably 1.2 to 1.8, more preferably 1.3 to 1.6, in particular 1.35 to 1.5.
(9) The process according to any one of items (1) to (8), wherein the proton donator is selected from the group consisting of inorganic or organic acids, preferably inorganic or organic acids having a pKₛ within a range of 3.0 to 6.0, more preferably ammonium salts, formic acid and acetic acid, even more preferably NH₄Cl, (NH₄)₂SO₄ and acetic acid, and in particular acetic acid.
(10) The process according to any one of items (1) to (9), wherein a first solvent and a second solvent are incorporated into the solvate form of a salt of compound of formula XI provided in step a) wherein the first solvent is different from the second solvent, preferably said first solvent is incorporated into the salt of compound of formula XI in an amount of percent by weight which is lower than the amount of percent by weight of the second solvent
The terms "first solvent"/"second solvent" as used herein denote the order in which said solvents are indicated within the chemical nomenclature used herein.
(11) The process according to item (10), wherein the compound of formula XI provided in step a) comprises said first solvent in an amount of about 0.1 to 8 percent by weight and said second solvent in an amount of about 3 to 15 percent by weight relative to the salt of compound of formula XI, preferably compound of formula XI provided in step a) comprises said first solvent in an amount of about 1 to 5 percent by weight and said second solvent in an amount of about 7.7 to 10 percent by weight relative to the salt of compound of formula XI.
(12) The process according to item (11), wherein said first solvent is an alcohol and said second solvent is a hydrocarbon or halocarbon, preferably said first organic solvent is a C₁-C₄-alcohol and said second organic solvent is a C₆-C₁₀-hydrocarbon comprising a benzene moiety or a C₁-C₁₀-halocarbon optionally comprising a benzene moiety, more preferably said first solvent is methanol or ethanol and said second solvent is toluene, methylene chloride, chloroform or chlorobenzene, in particular, said first solvent is methanol and said second solvent is methylene chloride.
(13) The process according to any one of items (1) to (12), wherein the salt or solvate form of a salt of compound of formula XI provided in step a) represents an acid addition salt of an acid selected from the group consisting of oxalic acid, succinic acid, lactic acid, malonic acid, HCl, HBr and H₂SO₄, more preferably HCl, HBr and H₂SO₄, even more preferably HCl and HBr, and in particular HBr.
(14) The process according to any one of items (1) to (13), wherein said hydrate of a compound of formula XI obtained by said process has a purity of > 98 %, preferably > 99 %, more preferably > 99.5 %.
According to this preferred embodiment, a highly pure intermediate is provided which represents a particularly suitable intermediate for the preparation of highly pure pharmaceutically acceptable salts of a compound of formula XI. Furthermore, the hydrate of this preferred embodiment may also be used directly as active agent in a pharmaceutical formulation.
(15) A hydrate of a compound of the formula XI' 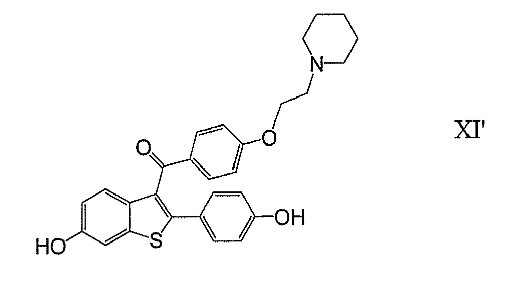 (16) The hydrate according to item (15), wherein said hydrate comprises 2.8 to 4.7 % by weight of water relative to a hydrate of a compound of formula XI, preferably 3.2 to 4.2 % by weight, more preferably 3.4 to 3.9 % by weight.
(17) The hydrate according to item (15) or (16), wherein said hydrate is free of antisolvent for a free base of a compound of formula XI' or preferably comprises said antisolvent in an amount of up to 15 % by weight relative to a hydrate of a compound of formula XI, more preferably up to 9.2 % by weight, even more preferably up to 7.4 % by weight and in particular 0.1 to 4.9 % by weight; preferably, said antisolvent is acetone.
As to the meaning of the term "free base" in items (16) and (17), reference is made to the explanation under item (1) above.
(18) The hydrate according to any one of items (15) to (17), wherein said hydrate is in crystalline form, preferably a hydrate in crystalline form whose (XRD) pattern has at least signals at the following 2θ values (±0.5° respectively):

| | | | | | | | | | | |
|---|---|---|---|---|---|---|---|---|---|---|
| **2theta[°]** | 8.13 | 13.13 | 15.18 | 18.11 | 18.39 | 18.65 | 23.07 | 23.49 | 23.76 | 26.02 |

, more preferably said hydrate in crystalline form exhibits an (XRD) pattern having at least signals at the following 2θ values (±0.5° respectively):

| | | | | | | | | | | |
|---|---|---|---|---|---|---|---|---|---|---|
| **2theta[°]** | 8.13 | 9.54 | 12.46 | 13.13 | 13.93 | 15.18 | 16.15 | 16.9 | 17.9 | 18.11 |
| | 18.39 | 18.65 | 19.44 | 20.63 | 21.84 | 22.08 | 22.43 | 23.07 | 23.49 | 23.76 |
| | 24.63 | 24.95 | 26.02 | 26.32 | 26.49 | 28.08 | 31.25 | 31.69 | 35.33 | 40.36 |

In this preferred embodiment, 2θ values may be exact or substantially correspond with a tolerance of ±0.2° or even ±0.5° with the given 2θ values.
(19) A crystalline solvate form of a compound of formula XI' 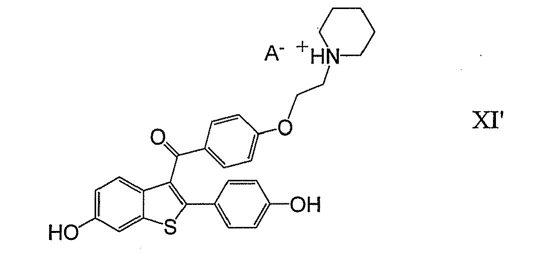 wherein A is the deprotonated form of an organic or inorganic acid, said crystalline solvate form comprising toluene as a solvent.
(20) A crystalline solvate form according to item (19), wherein said crystalline solvate form comprises toluene in an amount of about 0.1 to 15 percent by weight relative to the solvate form of a salt of compound of formula XI', preferably 3 to 12 percent by weight, more preferably 5 to 10 percent by weight and in particular 7 to 8.5 percent by weight.
(21) The crystalline solvate form according to item (19) or (20), wherein A is selected from the group consisting of oxalate, succinate, lactate, malonate, Cl⁻, Br⁻ and SO₄²⁻, preferably Cl⁻, Br⁻ and SO₄²⁻, more preferably Cl⁻, Br⁻, and even more preferably Br⁻.
(22) The crystalline solvate form according to any one of items (19) to (21), wherein A⁻ is Br⁻ and whose X-ray diffraction (XRD) pattern has at least signals at the following 2θ values (±0.5° respectively):

| | | | | | | | | | | |
|---|---|---|---|---|---|---|---|---|---|---|
| **2theta[°]** | 13.34 | 16.29 | 17.55 | 18.2 | 18.46 | 20.5 | 20.79 | 23.32 | 23.53 | 25.5 |

, preferably said crystalline solvate form exhibits an X-ray diffraction (XRD) pattern having at least signals at the following 2θ values (±0.5° respectively):

| | | | | | | | | | | |
|---|---|---|---|---|---|---|---|---|---|---|
| **2theta[°]** | 13.34 | 13.98 | 15.74 | 16.29 | 17.11 | 17.4 | 17.55 | 18.2 | 18.46 | 18.85 |
| | 18.97 | 19.23 | 19.61 | 20.22 | 20.5 | 20.79 | 21.1 | 22.59 | 23.32 | 23.53 |
| | 23.96 | 24.18 | 24.88 | 25.04 | 25.4 | 25.5 | 29.38 | 30.33 | 31.42 | 33.63 |

In this preferred embodiment, 2θ values may be exact or substantially correspond with a tolerance of ±0.2° or even ±0.5° with the given 2θ values.
(23) Use of a hydrate of a compound of formula XI 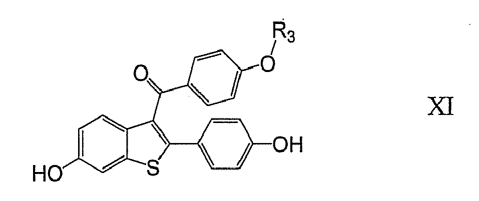 wherein R₃ is 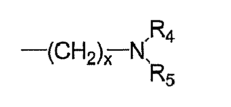 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring, preferably a 5- to 7-membered ring, more preferably a 5- or 6-membered ring, in particular a 6-membered ring,
in a process for preparing a pharmaceutically active agent; preferably said pharmaceutically active agent is compound of formula XI in form of raloxifene or derivatives thereof.
The term "pharmaceutically active agent" as used herein means any active pharmaceutical ingredient intended for treatment or prophylaxis of a disease of a mammal. In general it means any active pharmaceutical ingredient that has an effect on the physiological conditions of a mammal.
(24) The use according to item (23), wherein said hydrate of compound of formula XI comprises 2.8 to 4.7 % by weight of water relative to hydrate of a compound of formula XI, preferably 3.2 to 4.2 % by weight, more preferably 3.4 to 3.9 % by weight.
(25) The use according to item (23) or (24), wherein said hydrate comprises an antisolvent for a free base of a compound of formula XI' in an amount of up to 15 % by weight relative to a hydrate of a compound of formula XI, preferably up to 9.2 % by weight, more preferably up to 7.4 % by weight and in particular 0.1 to 4.9 % by weight; preferably, said antisolvent is acetone.
(26) The use according to any one of items (23) to (25), wherein the hydrate form of a compound of formula XI is a hydrate as defined in any one of items (16) to (19).
(27) A process for preparing a non-solvated pharmaceutically acceptable salt of a compound of formula XI 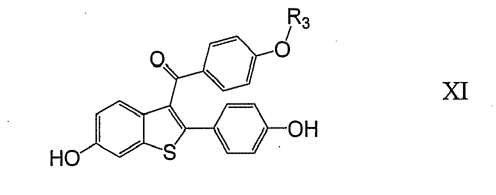 wherein R₃ is 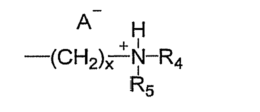 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring, preferably a 5- to 7-membered ring, more preferably a 5- or 6-membered ring, in particular a 6-membered ring,
wherein A⁻ is the deprotonated form of an organic or inorganic acid,
at least comprising the steps of:
i) providing a hydrate of a compound of formula XI 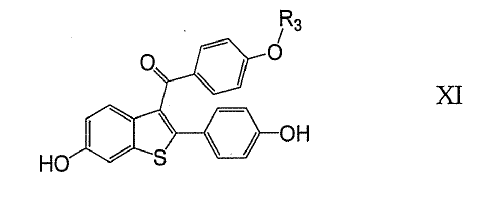 wherein R₃ is 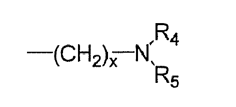
   , wherein x = 1 to 20, preferably 1 to 15, more preferably 2 to 10, and in particular 2,
   and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring, preferably a 5- to 7-membered ring, more preferably a 5- or 6-membered ring, in particular a 6-membered ring,
ii) dissolving said hydrate of step i) in organic solvent,
iii) adding a proton donor in order to quantitatively convert said hydrate of compound of formula XI into a pharmaceutically acceptable salt of a compound of formula XI,
iv) optionally seeding the mixture resulting from step v) in order to induce crystallisation,
v) allowing the pharmaceutically acceptable salt of compound of formula XI to precipitate, preferably to crystallize, optionally under agitation, and
vi) separating the pharmaceutically acceptable salt of a compound of formula XI, preferably separation is carried out by filtration.
As to the meaning of the terms "hydrate of a compound of formula XI","seeding" and "proton donor", reference is made to the explanations under item (1) above.
According to this preferred aspect of the invention, a process for preparing a non-solvated pharmaceutically acceptable salt of a compound of formula XI is provided, wherein the hydrate of a compound of formula XI is converted to the respective pharmaceutically acceptable salt in both high yields and exceptional purity, while the obtained pharmaceutically acceptable salt of a compound of formula XI is substantially free of water component and optional antisolvent component of the hydrate starting material.
(28) The process according to item (27), wherein said hydrate of compound of formula XI provided in step i) comprises 2.8 to 4.7 % by weight of water relative to hydrate of a compound of formula XI, preferably 3.2 to 4.2 % by weight, more preferably 3.4 to 3.9 % by weight.
(29) The process according to item (27) or (28), wherein said hydrate of compound of formula XI provided in step i) comprises an antisolvent for a free base of a compound of formula XI' in an amount of up to 15 % by weight relative to a hydrate of a compound of formula XI, preferably up to 9.2 % by weight, more preferably up to 7.4 % by weight and in particular 0.1 to 4.9 % by weight; preferably, said antisolvent is acetone.
As to the meaning of the term "antisolvent", reference is made to the explanations under item (1) above.
(30) The process according to any one of items (27) to (29), wherein the hydrate form of a compound of formula XI is a hydrate as defined in any one of items (16) to (19).
(31) The process according to any one of items (27) to (30), wherein in step ii), said organic solvent forms an azeotrope with the water and/or the optional antisolvent incorporated in said hydrate form, preferably the azeotrope in step ii) is formed under normal or reduced pressure, more preferably under reduced pressure.
The term "azeotrope" as used herein means a mixture of two (or more) fluid components, in this case water and optionally antisolvent)/organic solvent applied in step ii), wherein said two (or more) fluid components can not be seperated by simple distillation, that is distillation without rectification step.
(32) The process according to any one of items (27) to (31), wherein in step iii), the resulting mixture is heated prior to step iv), preferably heating is under reflux conditions.
(33) The process according to item (31) or (32), wherein the water and the optional antisolvent incorporated into said hydrate of formula XI is/are removed by azeotropic distillation of the mixture resulting from step ii), preferably said azeotropic distillation is carried out at reduced pressure.
(34) The process according to any one of items (27) to (33), wherein said organic solvent of step ii) is an alcohol, preferably a water-miscible alcohol, more preferably a C₁-C₄-alcohol, even more preferably methanol or ethanol, and in particular methanol.
(35) The process according to any one of item (27) to (34), wherein said organic solvent of step ii) is free of water or preferably comprises a predetermined amount of water, more preferably said amount of water is predetermined by a volume ratio of water and organic solvent of up to 0.5, even more preferably 0.24 to 0.44, yet even more preferably 0.27 to 0.40, and in particular 0.29 to 0.38.
(36) The process according to any one of items (27) to (35), wherein said water used in step ii) is potable water, preferably deionized water, more preferably reverse osmosis water.
(37) The process according to any one of items (27) to (36), wherein said proton donor used in step iii) is selected from the group consisting of oxalic acid, succinic acid, lactic acid, malonic acid, HCl, HBr and H₂SO₄, more preferably HCl, HBr and H₂SO₄, even more preferably HCl and HBr, and in particular HBr.
(38) The process according to any one of items (27) to (37), wherein activated charcoal is added in step iii) and said charcoal is separated from the mixture resulting from step iii) prior to step iv), preferably separation is carried out by filtration.
(40) The process according to items (1) to (14), wherein the compound of formula XI provided in step a) is prepared via a process for preparing a compound of formula VII' 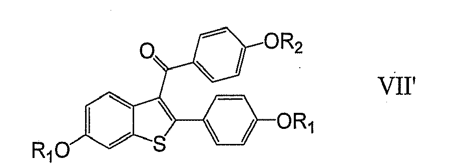 wherein R₁ and R₂ represent different hydroxy protecting groups which are independently from each other selectively removable, with the proviso that both -OR₁ and -OR₂ are substantially not cleavable in the presence of a Lewis acid at Friedel-Crafts acylation conditions,
wherein a compound of formula VI' 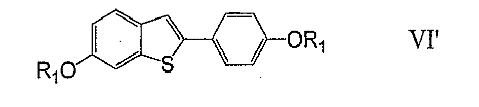 is reacted with a compound of formula V 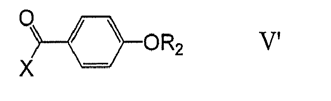 wherein R₁ and R₂ are defined as above, and X represents Cl, Br, I or 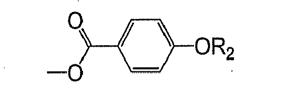 wherein R₂ is defined as above,
in the presence of a Lewis acid and a solvent.
The term "hydroxy protecting group" as used herein means any group known in the art which can be used for protecting a hydroxy group, with the proviso that the cleavage conditions for selectively removing said hydroxy protecting group will not adversely affect the structure of compounds of formula VII, VII', VIII or X.
As to the meaning of the terms "alkyl", "aryl", "alkylaryl" and "substituted", reference is made to the explanations under item (1) above.
The term "substantially not cleavable in the presence of a Lewis acid at Friedel-Crafts acylation conditions" as used herein means that there is no or substantially no deprotection of -OR₁ and -OR₂ at Friedel-Crafts acylation conditions. In a suitable "Friedel-Crafts acylation condition" for testing absence of cleavage, a Lewis acid, that is an electron acceptor, which acts as a catalyst, may be used for example in about 1 molar amount to about 2 molar amount relative to the compound to be acylated, wherein the temperature of the reaction mixture is typically within a range of about 0°C to ambient temperature.
According to this embodiment of the invention, the starting materials compound of formula VI' and compound of formula V are suitably selected in order to obtain highly desirable products of formula VII'. Compound of formula VII' represents a useful intermediate for preparing pharmaceutically active agents such as raloxifene or derivatives thereof, since the introduction of the two different protecting groups R₁ and R₂ provides for an advantageous selective derivatisation of compound of formula VII' in subsequent reaction steps.
Furthermore, by suitably selecting R₁ and R₂, the possibly different cleavability/bond strength of O-R₁ and O-R₂ for a certain protecting group at the 2- and 6-positions compared to the 3-position of the benzo[b]thiophene moiety can be taken into account.
(41) The process according to item (40), wherein one of the hydroxy protecting groups R₁ and R₂ is stable under basic conditions and cleavable under acidic conditions but substantially not cleavable in the presence of a Lewis acid at Friedel-Crafts acylation conditions, while the other hydroxy protecting group is cleavable under basic conditions and stable under acidic conditions; preferably, R₁ is stable under basic conditions and cleavable under acidic conditions but substantially not cleavable in the presence of a Lewis acid at Friedel-Crafts acylation conditions, and R₂ is cleavable under basic conditions and stable under acidic conditions.
The term "basic conditions" as used herein means conditions wherein Brønsted bases, that is proton acceptors, are applied and added.
The term "acidic conditions" as used herein means conditions wherein Brønsted acids, that is proton donators, or Lewis acids, that is electron acceptors, are applied and added.
Besides the type of acid or base used, the reaction conditions of said "basic conditions" and "acidic conditions" have to be suitably selected such that there is no undesired cleavage due to harsh reaction conditions like large excess of acid or base, high reaction temperatures and/or long reaction times. For Lewis acids for example, cleavage of the protecting group(s) due to the presence of Lewis acid is undesired in the acylation process according to item (40), while cleavage by a Lewis acid may be carried out under suitable reaction conditions in a reaction step subsequent to acylation.
This measure provides for an efficient and economical deprotection of R₁ and R₂ respectively, since Brønsted bases and acids as well as Lewis acids are readily available and economic reactants for cleavage.
(42) The process according to item (40) or (41), wherein R₁ is selected from the group consisting of linear or branched alkyl, linear or branched alkylaryl and linear or branched alkenyl; preferably R₁ is linear or branched alkyl;
and R₂ is selected from the group consisting of linear or branched sulfonylalkyl, sulfonylaryl and linear or branched sulfonylalkylaryl; preferably R₂ is sulfonylaryl; or
(43) The process according to any one of items (40) to (42), wherein R₁ is selected from the group consisting of methyl, ethyl, tert-butyl, benzyl, p-methoxybenzyl (PMB), trityl (Tr), allyl; preferably methyl, ethyl, tert-butyl, more preferably methyl;
and R₂ is selected from the group consisting of mesyl, tosyl and benzene sulfonyl; preferably R₂ is tosyl and benzene sulfonyl; more preferably R₂ is benzene sulfonyl.
(44) The process according to any one of items (40) to (43), wherein R₁ is methyl and R₂ is benzene sulfonyl.
(45) The process according to any one of items (40) to (44), wherein X is Cl.
(46) The process according to any one of items (40) to (45), wherein the Lewis acid is used in substoichiometric amounts relative to compound of formula VI and VI' respectively, preferably in about 0.004 to 0.07 times molar amounts, more preferably in about 0.008 to 0.05 times molar amounts, and in particular in an amount of 0.01 to 0.02 relative to compound of formula VI and VI' respectively.
According to this beneficial embodiment of the invention, the amount of Lewis acid as a catalyst of the Friedel-Crafts acylation can be significantly reduced compared to conventional Friedel-Crafts conditions, especially those conventionally applied to similar reactants, wherein an excess of Lewis acid relative to the compound to be acylated is used. Thereby, acylation of compound of formula VI or VI' can be carried out under mild reaction conditions, which in turn provides for a stable and reliably process, since cleavage of the OR₁ moieties is avoided owing to the significantly reduced amount of Friedel-Crafts catalyst. Thus, this embodiment provides for high yields and less require efforts for purifying the product, while said process is also advantageous from economical view due to the substantial savings of Lewis acid catalyst.
(47) The process according to any one of items (40) to (46), wherein the Lewis acid comprises divalent or trivalent metal, preferably divalent metal, more preferably divalent metal generated in situ by comproportionation of elemental metal and salt comprising trivalent metal.
In this way, the Lewis acid catalyst is suitably selected in view of the activity of the catalyst. That is, the above defined Lewis acids provide for both fast reaction rates and high conversion rates. By suitably selecting the valency of the metal comprised in the Lewis acid, activity of the Lewis acid catalyst can be improved. Furthermore, activity of the Lewis acid catalyst may be improved by generating the metal comprised in the catalyst in situ.
(48) The process according to any one of items (40) to (47), wherein the Lewis acid is FeCl₃, FeCl₂, FeBr₃, FeBr₂, FeSO₄, Fe₂(SO₄)₃, ZnCl₂, ZnBr₂, ZnSO₄; preferably FeCl₂, FeSO₄, ZnCl₂; more preferably FeSO₄, ZnCl₂; even more preferably FeSO₄ generated in situ by comproportionation of elemental Fe and Fe₂(SO₄)₃, and in particular FeSO₄ generated in situ by comproportionation of elemental Fe and Fe₂(SO₄)_{3·}5H₂O. According to this embodiment of the invention, both metal cation and counter anion are suitably selected with the proviso that the Lewis acid catalyst has an improved activity.
(49) The process according to any one of items (40) to (48), wherein chlorobenzene, dichlorobenzene or toluene is used as the solvent, preferably chlorobenzene or toluene, more preferably toluene.
This embodiment of the invention provides for improvement of environmental friendliness of the process, while there is also an improvement in view of the working conditions. Under conventional Friedel-Crafts acylation conditions, halocarbons such as dichloroethane or methylene chloride are used as the solvent, wherein said halocarbons are hazardous to health and exhibit low boiling points. In contrast to that, toluene has a high boiling point and is much less hazardous to health. Toluene can not be used as a solvent in conventional Friedel-Crafts acylations, since toluene would be acylated, too. In contrast to that, under the process conditions defined in item (46) to (48) above, there is substantially no acylation of toluene as the solvent.

### Detailed description of the invention

The present invention is now described in more detail by referring to further preferred and further advantageous embodiments and examples, which are however presented for illustrative purposes only and shall not be understood as limiting the scope of the present invention.

According to one aspect of the invention, a crystalline hydrate of a compound of formula XI 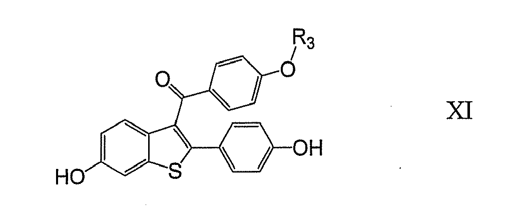 wherein R₃ is 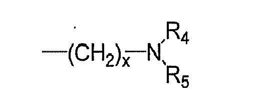 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring, preferably a 5- to 7- membered ring, more preferably a 5- or 6-membered ring, in particular a 6-membered ring,
is prepared by a process comprising the steps of:
a) providing a free base of the compound of formula XI, a salt of the compound of formula XI or a solvate form of a salt of the compound of formula XI comprising at least one solvated organic solvent,
b) dissolving said compound provided in step a) with a mixture of an antisolvent for a free base of a compound of formula XI and an aqueous solution of a proton acceptor in an at least stoichiometric molar amount or higher relative to the compound of formula XI provided in step a),
c) adjusting the pH of the mixture resulting from step b) to 7-9 by adding a proton donator,
d) optionally seeding the mixture resulting from step c) in order to induce crystallisation,
e) allowing hydrate of compound of formula XI to precipitate, preferably to crystallize, optionally under agitation, and
f) separating the crystalline hydrate of compound of formula XI obtained in step e), preferably separation is carried out by filtration.

It was surprisingly found that the above procedural concept provides a hydrate of a compound of formula XI. Said hydrate of a compound of formula XI represents a highly valuable intermediate for the preparation of pharmaceutically active agents such as raloxifene or derivatives thereof, since the formation of the hydrate form of compound of formula XI provides for a highly effective purification step in the synthetic path for preparing a compound of formula XI. Furthermore, the present process for preparing a hydrate of a compound of formula XI is universally applicable to a free base, a salt or a solvate form of a salt of a compound of formula XI and a solvate form of a salt of a compound of formula XI. The hydrate of the compound of formula XI obtained in step f) comprises compound of formula XI in form of its free base wherein the nitrogen atom of R₃ is not protonated. Thus, said hydrate furthermore represents a versatile compound for producing a pharmaceutically composition due to its high purity, it may be used either directly or merely further converted into a pharmaceutically acceptable salt for the purpose of formulating a pharmaceutical composition.

Preferably, the hydrate of a compound of formula XI comprises 2.8 to 4.7 % by weight of water relative to a hydrate of a compound of formula XI, preferably 3.2 to 4.2 % by weight, more preferably 3.4 to 3.9 % by weight.

In the above described process for preparing a crystalline hydrate of a compound of formula XI, acetone is preferably used as an antisolvent for a free base of a compound of formula XI. Acetone is the preferred antisolvent, since it provides for an effective and quantitative precipitation or crystallisation of the hydrate of a compound of formula XI.

The hydrate of compound of formula XI preferably is free of said antisolvent or more preferably comprises said antisolvent in an amount of up to 15 % by weight relative to a hydrate of a compound of formula XI, even more preferably up to 9.2 % by weight, yet even more preferably up to 7.4 % by weight and in particular 0.1 to 4.9 % by weight.

According to one embodiment of the invention, the obtained crystalline hydrate of a compound of formula XI is a hydrate of raloxifene with the formula 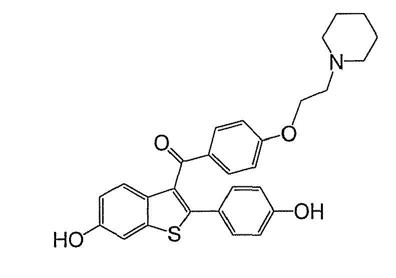 , preferably said hydrate of raloxifene exhibits an X-ray diffraction (XRD) pattern having at least signals at the following 2θ values (± 0.5° respectively):

| | | | | | | | | | | |
|---|---|---|---|---|---|---|---|---|---|---|
| **2theta[°]** | 8.13 | 13.13 | 15.18 | 18.11 | 18.39 | 18.65 | 23.07 | 23.49 | 23.76 | 26.02 |

, more preferably said hydrate of raloxifene exhibits an X-ray diffraction (XRD) pattern having at least signals at the following 2θ values (± 0.5° respectively):

| | | | | | | | | | | |
|---|---|---|---|---|---|---|---|---|---|---|
| **2theta[°]** | 8.13 | 9.54 | 12.46 | 13.13 | 13.93 | 15.18 | 16.15 | 16.9 | 17.9 | 18.11 |
| | 18.39 | 18.65 | 19.44 | 20.63 | 21.84 | 22.08 | 22.43 | 23.07 | 23.49 | 23.76 |
| | 24.63 | 24.95 | 26.02 | 26.32 | 26.49 | 28.08 | 31.25 | 31.69 | 35.33 | 40.36 |

According to another embodiment of the invention, the proton acceptor is selected from the group consisting of inorganic bases or organic amine bases, preferably alkaline metal hydroxides, earth alkaline metal hydroxides, ammonia and triethylamine, more preferably alkaline metal hydroxides, even more preferably sodium hydroxide and potassium hydroxide, and in particular sodium hydroxide. Preferably, said at least stoichiometric amount of proton acceptor applied in step b) is 1 to 10 molar equivalents relative to the compound of formula XI provided in step a), more preferably 1.5 to 8 molar equivalents, even more preferably 3 to 7 molar equivalents and in particular 4.2 to 6 molar equivalents. According to a further preferred embodiment, the volume ratio of antisolvent for a free base of a compound of formula XI to aqueous proton acceptor of the mixture of step b) is in range from 1 to 2, preferably 1.2 to 1.8, more preferably 1.3 to 1.6, in particular 1.35 to 1.5.
In this way, by suitably selecting the kind and amount of proton acceptor as well as the volume ratio of antisolvent for a free base of a compound of formula XI to aqueous proton acceptor, beneficial conditions can be set for step b) which contribute to a complete dissolution of the compound provided in step a).

Said proton donator used in step c) is preferably selected from the group consisting of inorganic or organic acids, preferably inorganic or organic acids having a pKₛ within a range of 3.0 to 6.0, more preferably ammonium salts, formic acid and acetic acid, even more preferably NH₄Cl, (NH₄)₂SO₄ and acetic acid, and in particular acetic acid. The aforementioned proton donators represent relatively weak acids. Thus, when added to the reaction mixture, they will provide for a buffer characteristic of the reaction mixture of step c). Advantageously, said buffer characteristic provides for a simplified adjustment of the pH of the reaction mixture in step c).

A solvate form of a salt of a compound of formula XI is preferably provided as a starting material in step a). Said solvate form incorporates a first solvent and a second solvent, wherein the first solvent is different from the second solvent. Preferably, said first solvent is incorporated into the salt of compound of formula XI in an amount of percent by weight which is lower than the amount of percent by weight of the second solvent. More preferably, said solvate form of a compound of formula XI comprises said first solvent in an amount of about 0.1 to 8 percent by weight and said second solvent in an amount of about 3 to 15 percent by weight relative to the salt of compound of formula XI. Even more preferably, said solvate form of a compound of formula XI comprises said first solvent in an amount of about 1 to 5 percent by weight and said second solvent in an amount of about 7.7 to 10 percent by weight relative to the salt of compound of formula XI.
Preferably, said first solvent and said second solvent incorporated into said solvate form of a salt of a compound of formula XI are selected as follows: said first solvent is an alcohol and said second solvent is a hydrocarbon or halocarbon, preferably said first organic solvent is a C₁-C₄-alcohol and said second organic solvent is a C₆-C₁₀-hydrocarbon comprising a benzene moiety or a C₁-C₁₀halocarbon optionally comprising a benzene moiety, more preferably said first solvent is methanol or ethanol and said second solvent is toluene, methylene chloride, chloroform or chlorobenzene, in particular, said first solvent is methanol and said second solvent is methylene chloride.

In this way, a particularly suitable starting material is provided, since properties of a solvate form, such as melting point, chemical reactivity, density and solubility can be suitably set by incorporating the aforementioned first and second solvent into a salt of a compound of formula XI. Setting the aforementioned properties in turn provides for a suitable setting of procedural conditions in a process for preparing a hydrate of a compound of formula XI.

According to another embodiment of the invention, the salt or solvate form of a salt of compound of formula XI provided in step a) represents an acid addition salt of an acid selected from the group consisting of oxalic acid, succinic acid, lactic acid, malonic acid, HCl, HBr and H₂SO₄, more preferably HCl, HBr and H₂SO₄, even more preferably HCl and HBr, and in particular HBr. The aforementioned acids provide for a suitable setting of the chemical and/or physical properties of the starting material in view of procedural conditions in a process for preparing a hydrate of a compound of formula XI.

Said hydrate of a compound of formula XI obtained in step f) preferably has a purity of > 98 %, more preferably > 99 %, even more preferably > 99.5 %. In this way, a hydrate of a compound of formula XI is obtained in exceptional high purity. Thus, said hydrate represents a highly valuable intermediate for the preparation of pharmaceutically active agents such as raloxifene or derivatives thereof. Furthermore, such a highly pure hydrate may also be used directly as active agent in a pharmaceutical formulation.

According to another aspect of the invention, a process for preparing a non-solvated pharmaceutically acceptable salt of a compound of formula XI 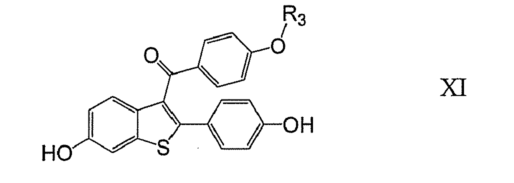 wherein R₃ is 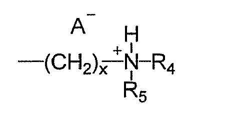
, wherein x = 1 to 20, preferably 1 to 15, more preferably 2 to 10, and in particular 2,
and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring, preferably a 5- to 7-membered ring, more preferably a 5- or 6-membered ring, in particular a 6-membered ring,
wherein A⁻ is the deprotonated form of an organic or inorganic acid, is prepared by a process comprising the steps of:
   i) providing a hydrate of a compound of formula XI 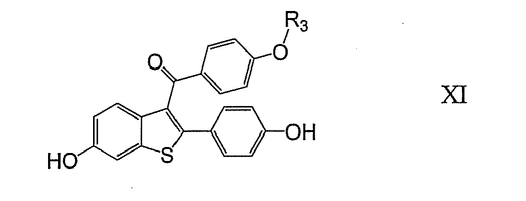 wherein R₃ is 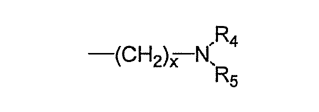 , wherein x = 1 to 20, preferably 1 to 15, more preferably 2 to 10, and in particular 2, and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring, preferably a 5- to 7-membered ring, more preferably a 5- or 6-membered ring, in particular a 6-membered ring,
   ii) dissolving said hydrate of step i) in organic solvent,
   iii) adding a proton donor in order to quantitatively convert said hydrate of compound of formula XI into a pharmaceutically acceptable salt of a compound of formula XI,
   iv) optionally seeding the mixture resulting from step v) in order to induce crystallisation,
   v) allowing the pharmaceutically acceptable salt of compound of formula XI to precipitate, preferably to crystallize, optionally under agitation, and
   vi) separating the pharmaceutically acceptable salt of a compound of formula XI, preferably separation is carried out by filtration.

The above mentioned aspect provides a non-solvated pharmaceutically acceptable salt of a compound of formula XI, wherein the hydrate of a compound of formula XI is converted to the respective pharmaceutically acceptable salt in both high yields and exceptional purity, while the obtained pharmaceutically acceptable salt of a compound of formula XI is substantially free of water component and optional antisolvent component of the hydrate starting material.

Said hydrate of a compound of formula XI is preferably a hydrate obtained by the above described process for preparing a crystalline hydrate of a compound of formula XI.

Preferably, in step ii), said organic solvent forms an azeotrope with the water and/or the optional antisolvent incorporated in said hydrate form, more preferably, said azeotrope in step ii) is formed under normal or reduced pressure, even more preferably under reduced pressure.

According to one embodiment of the invention, in step iii), the resulting mixture is heated prior to step iv), preferably heating is under reflux conditions.

According to a further embodiment of the invention, the water and/or the optional antisolvent incorporated into said hydrate of a compound of formula XI is/are removed by azeotropic distillation of the mixture resulting from step ii), preferably said azeotropic distillation is carried out at reduced pressure.
Said organic solvent applied in step ii) is preferably an alcohol, more preferably a water-miscible alcohol, even more preferably a C₁-C₄-alcohol, yet even more preferably methanol or ethanol, and in particular methanol. According to a further preferred embodiment, said organic solvent of step ii) is free of water or preferably comprises a predetermined amount of water, more preferably said amount of water is predetermined by a volume ratio of water and organic solvent of up to 0.5, even more preferably 0.24 to 0.44, yet even more preferably 0.27 to 0.40, and in particular 0.29 to 0.38.
Preferably, said water used in step ii) is potable water, more preferably deionized water, and in particular reverse osmosis water.
Furthermore, said proton donor used in step iii) is preferably selected from the group consisting of oxalic acid, succinic acid, lactic acid, malonic acid, HCl, HBr and H₂SO₄, more preferably HCl, HBr and H₂SO₄, even more preferably HCl and HBr, and in particular HBr. Preferably, said pharmaceutically acceptable salt of a compound of formula XI has a purity of > 99% measured by HPLC, preferably > 99.2%, more preferably > 99.4%, in particular > 99.6%.

Reaction Scheme 1 illustrates a preferred synthesis pathway for preparing a compound of formula XI: 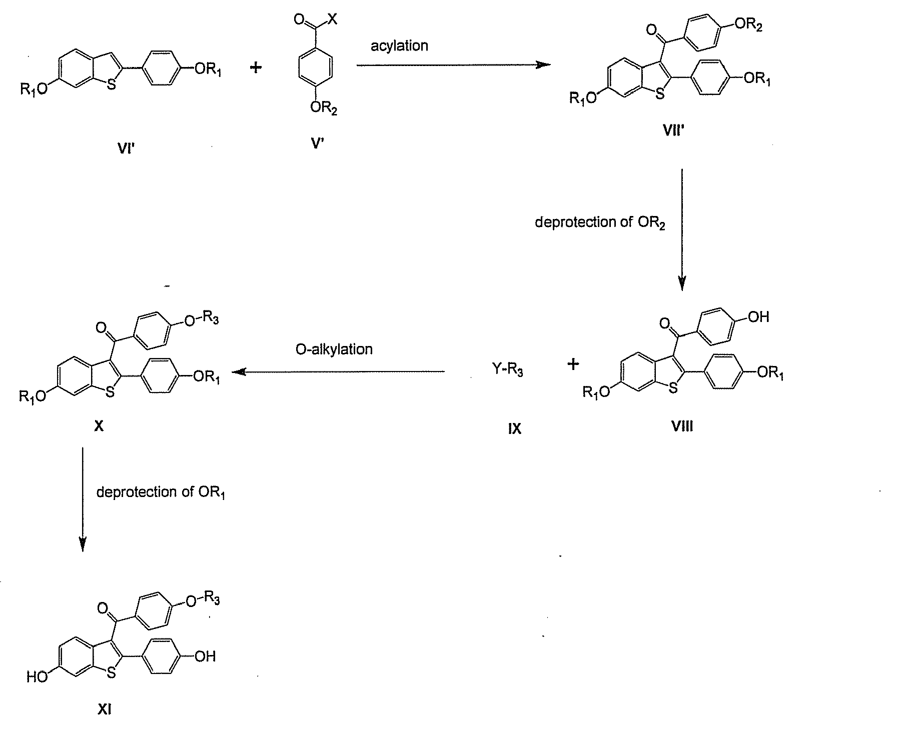 Scheme 1

According to the preferred embodiment of Scheme 1 (wherein R₁, R₂, R₃, X and Y are defined as in the preceding items), a compound of formula VII' is prepared by acylating a compound of formula VI' with a compound of formula V in the presence of a Lewis acid catalyst.

Compound of formula V' is preferably prepared by a synthesis pathway as depicted in Scheme 2 below. 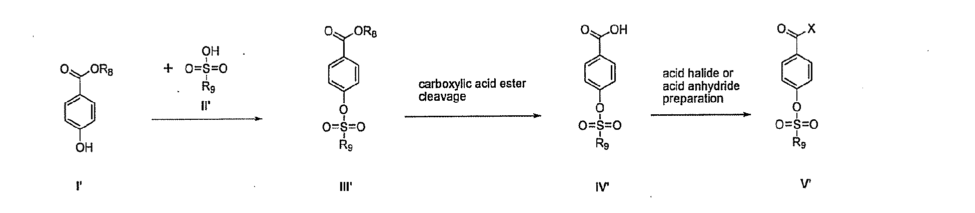 Scheme 2

According to the preferred embodiment of Scheme 2 (wherein R₈ and R₉ are defined as in the preceding items), a compound of formula V' is prepared by esterification of a compound of formula I' with a compound of formula II'. Next, compound of formula III' is converted into compound of formula IV' by cleavage of the carboxylic acid ester moiety, and compound of formula IV' in turn is converted to a carboxylic acid halogenide of formula V'. For example, compound of formula V may be obtained by using the following reactants: thionyl chloride or phosphorus pentachloride for obtaining carboxylic acid chlorides and phosphorus pentabromide for obtaining carboxylic acid bromides respectively.

Compound of formula VI' is readily available. For example, it can be prepared by reacting m-methoxythiophenol with α-bromo-4-methoxyacetophenone as described in US 4,133,814.

The advantage of introducing two different protecting groups R₁ and R₂ into compound of formula VII' is that an orthogonal protecting group strategy is provided, that is R₁ and R₂ can be independently from each other selectively removed. Protecting groups R₁ and R₂ have to be suitably selected with the proviso that -OR₁ and -OR₂ are substantially not cleavable in the presence of a Lewis acid at Friedel-Crafts acylation conditions.

Conventionally, Friedel-Crafts acylation is carried out with a Lewis acid as the catalyst in about 1 molar amount to about 2 molar amount relative to the compound to be acylated, wherein the temperature of the reaction mixture is typically within a range of about 0°C to ambient temperature, wherein elevated temperatures may be required for less reactive compounds to be acylated.

It was surprisingly found that Friedel-Crafts acylation can be carried out with Lewis acid catalyst used in substoichiometric amounts relative to the compound to be acylated, wherein the temperature of the reaction mixture is within a range of about 50°C to reflux temperature of the reaction mixture. Preferably, the Lewis acid catalyst is used in about 0.004 to 0.07 times molar amounts, more preferably in about 0.008 to 0.05 times molar amounts, and in particular in an amount of 0.01 to 0.02 relative to the compound to be acylated. Since the amount of Lewis acid as the catalyst can be significantly reduced compared to conventional Friedel-Crafts conditions, acylation of compound of formula VI' can be carried out under mild reaction conditions. Thus, a stable and reliably process is provided, since cleavage of the OR₁ moieties is avoided owing to the significantly reduced amount of Friedel-Crafts catalyst.

Mostly, Lewis acids such as BCl₃, BF₃ and AlCl₃ are used as a catalyst for conventional Friedel-Crafts acylation. Surprisingly, Lewis acids comprising divalent metal represent effective catalysts for Friedel-Crafts acylation providing both fast reaction rates and high conversion rates. Preferably, a divalent metal generated in situ by comproportionation of elemental metal and salt comprising trivalent metal is used as the Lewis acid catalyst.

Furthermore, both metal cation and counter anion may be suitably selected in order to improve activity of the Lewis acid catalyst. Preferably, the Lewis acid is FeCl₃, FeCl₂, FeBr₃, FeBr₂, FeSO₄, Fe₂(SO₄)₃, ZnCl₂, ZnBr₂, ZnSO₄; more preferably FeCl₂, FeSO₄, ZnCl₂; even more preferably FeSO₄, ZnCl₂; in particular FeSO₄ generated in situ by comproportionation of elemental Fe and Fe₂(SO₄)₃, wherein Fe₂(SO₄)3 is preferably applied in pentahydrate form. Surprisingly, Fe₂(SO₄)₃ in pentahydrate form represents an effective catalyst, since under conventional Friedel-Crafts acylation conditions, water disturbs or even prevents the acylation reaction.

Preferably, chlorobenzene, dichlorobenzene or toluene is used as the solvent in the acylation step, more preferably chlorobenzene or toluene, and in particular toluene. Conventionally, at Friedel-Crafts acylation conditions, halocarbons such as dichloroethane or methylene chloride are preferably used as the solvent. However, such halocarbons are hazardous to health. Furthermore, they exhibit low boiling points, that is they are relatively volatile. Therefore, it is very likely that maximum allowable concentration (MAC) values may be exceeded. The aromatic solvents chlorobenzene, dichlorobenzene or toluene provide for efficient reaction rates. Furthermore, the relatively high boiling aromatics chlorobenzene, dichlorobenzene and toluene are less hazardous than the aforementioned halocarbons. Surprisingly, chlorobenzene, dichlorobenzene and toluene provide for an efficient acylation, while further providing improvement of both environmental friendliness of the process and working conditions. Even more surprisingly, it was found that toluene represents a suitable solvent for Friedel-Crafts acylation, since toluene is acylated under conventional Friedel-Crafts conditions and can therefore not be used as a solvent under conventional Friedel-Crafts conditions.

Further according to the preferred embodiment illustrated by Scheme 1, a compound of VIII is prepared by subjecting a compound of formula VII' to a deprotection reaction, wherein -OR₂ is selectively cleaved. Said selective deprotection reaction is enabled by the beneficial present orthogonal protecting group strategy, i.e. introducing two different hydroxy protecting groups R₁ and R₂ in compound of formula VII' by the aforementioned acylation step.

The hydroxy protecting groups R₁ and R₂ are stable under basic conditions and cleavable under acidic conditions, but substantially not cleavable in the presence of a Lewis acid at Friedel-Crafts acylation conditions, while the other hydroxy protecting group is cleavable under basic conditions and stable under acidic conditions. Preferably, R₁ is stable under basic conditions and cleavable under acidic conditions but substantially not cleavable in the presence of a Lewis acid at Friedel-Crafts acylation conditions, and R₂ is cleavable under basic conditions and stable under acidic conditions. More preferably R₁ is selected from the group consisting of linear or branched alkyl, linear or branched alkylaryl and linear or branched alkenyl; preferably R₁ is linear or branched alkyl, and R₂ is selected from the group consisting of linear or branched sulfonylalkyl, sulfonylaryl and linear or branched sulfonylalkylaryl; preferably R₂ is sulfonylaryl. Even more preferably, R₁ is selected from the group consisting of methyl, ethyl, tert-butyl, benzyl, p-methoxybenzyl (PMB), trityl (Tr), allyl; preferably methyl, ethyl, tert-butyl, more preferably methyl, and R₂ is selected from the group consisting of mesyl, tosyl and benzene sulfonyl; preferably R₂ is tosyl and benzene sulfonyl; more preferably R₂ is benzene sulfonyl. In particular, R₁ is methyl and R₂ is benzene sulfonyl.

By suitably selecting protecting groups R₁ and R₂, which are located at different positions of the benzo[b]thiophene moiety, complete deprotection of both -OR₁ and -OR₂ can be attained under mild conditions. It is known that cleavability of a certain protecting group, for example methyl, may differ at the different positions of the benzo[b]thiophene moiety. For example, WO 98/48792, which does not disclose an orthogonal protecting group strategy since all hydroxy protecting groups are methyl groups, describes that a methoxy group at the 3-benzoyl substituent of the benzo[b]thiophene moiety can not be cleaved under conventional cleavage conditions wherein a large excess of Lewis acid such as BCl₃ is applied as the cleaving agent, while methoxy groups at the 2- and 6-positions of benzo[b]thiophene are smoothly cleaved under the aforementioned conditions. For removing the methyl group from the methoxy group at the 3-benzoyl substituent of the benzo[b]thiophene moiety, harsh conditions like refluxing in pyridinium chloride had to be applied, nevertheless removal was not complete.

Preferably, the aforementioned deprotection step for cleavage of the -OR₂ group is carried out under phase transfer catalysis conditions, wherein the reaction is carried out in a mixture of organic solvent such as toluene and water in the presence of an inorganic base such as NaOH or KOH and a phase transfer catalyst such as tetra-n-butylammonium bromide (TBAB).

Subsequent to deprotection of -OR₂, compound of formula VIII is converted to compound of formula X by O-alkylation using compound of formula IX as the alkylation reagent. Preferably, Y of the alkylation reagent is Cl. Furthermore, R₃ preferably has the following formula 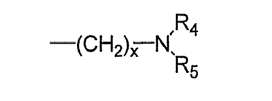
, wherein x = 1 to 20, preferably 1 to 15, more preferably 2 to 10, and in particular 2,
and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring, preferably a 5- to 7-membered ring, more preferably a 5- or 6-membered ring, in particular a 6-membered ring. Further according to the preferred embodiment illustrated by Scheme 1, subsequent to O-alkylation, a deprotection reaction is carried out in order to convert compound of formula X to compound of formula XI. For example, in case R₁ of compound of formula X is alkyl such as methyl, deprotection of the -OR₁ groups is carried out with a Lewis acid such as BCl₃ as the cleaving agent. Since -OR₁ is substantially not cleavable in the presence of a Lewis acid at Friedel-Crafts acylation conditions, deprotection conditions have to be suitably selected in order to achieve complete cleavage of -OR₁. Therefore, the Lewis acid as the cleavage agent is used in relatively large excess, preferably in 2 to 7 times molar amounts, preferably 2.4 to 6 times molar amounts; more preferably 2.6 to 5 times molar amounts and in particular 2.9 to 4.5 times molar amounts relative to compound of formula X.
Preferably, compound of formula XI represents a pharmaceutically active agent such as raloxifene.

The following examples are merely illustrative of the present invention and they should not be considered as limiting the scope of the invention in any way. The examples and modifications or other equivalents thereof will become apparent to those versed in the art in the light of the present entire disclosure.

### Examples

### Methods

**Heavy metals.** According to USP (231, method II). **Sulphated ash.** According to Ph. Eur. (2.4.14). **IR.** FTIR-spectrometer Spectrum one (Perkin-Elmer); according to Ph. Eur. (2.2.24, KBr). **DSC.** DSC822 (Mettler), inert gas: nitrogen, temperature program from 30°C to 400°C **/** heating rate: 10°C/min. **Water content.** Titrando 835 (Metrohm) with double-Pt-electrode; Karl-Fisher-titration (abbreviated as KFT in the following Examples). **MP.** B545 (Büchi). **Residual solvents.** Gas chromatograph 6890 (Agilent) with head space sampler; head space gas chromatography, column DB-624: 30 m length, 0.32 mm ID, 1,8 µm film thickness, temperature program from 40°C to 250°C. **Titration.** Titrino 716 / Titrando 835 (Metrohm); with sodium hydroxide solution and solvotrode, with silver nitrate solution and silver titrode. **NMR.** Bruker AVANCE 300 spectrometer at 301 K. **XRPD.** transmission diffractometer STADI P (STOE & Cie., Darmstadt, Germany) using Ge-monochromated Cukα₁ radiation (1.5406 A) in Debye-Scherrer-geometry; the most intense 2θ-peaks of the obtained X-ray diffractograms were determined by the analysis software "Match! Version 1.9d, Crystal Impact, 2003-2009".

**Gas Chromatography.** The analysis of **compounds of formula VII' was** carried out by using an GC-system consisting of a split/splitless-injector, an autosampler, column oven and FI-detector (Agilent), using a Rxi-5-MS column (25 m x 0.25 mm ID, 0.5 µm film thickness). Injection volume 1 µl. Heating rate 10 K/min.

| | |
|---|---|
| Hydrogen | 40 ml/min |
| Air | 450 ml/min |
| Make-up gas (N₂) | 25 ml/min |

A sample (50 mg) was dissolved in 1.5 mL of toluene and assayed by gas chromatography.

| | |
|---|---|
| Carrier gas, flow rate | Nitrogen: 1.0 mL/min, constant flow |
| Split flow rate | 20 mL/min |
| Injector temperature | 330°C |
| Oven temperature program: | |
| Start temperature/holding time | 50°C / 0 min |
| Final temperature/holding time | 330°C / 60 min |
| Duration of analysis | 88 min |
| Detector / temperature | 330°C |

**GC-MS.** The analysis of **compounds of formula VII'** was carried out by using an GC-MS-system consisting of a split/splitless-injector; an autosampler, column oven and MSD-detector (Agilent), using a Rxi-5-MS column (25 m x 0.25 mm ID, 0.5 µm film thickness). Carrier gas, flow rate helium: 1.9 mL/min, constant flow. Split flow rate 38 mL/min. Oven temperature program: Start temperature/holding time 50°C / 0 min. Heating rate 10 K/min. Injection volume 1 µl.

| | |
|---|---|
| AUX / temperature | 350°C |
| MS Source / temperature | 230°C |
| MS Quad / temperature | 150°C |
| Scan masses | from 15 to 550 |
| Threshold | 25 |

A sample (50 mg) was dissolved in 1 mL of toluene and subjected to GC/MS analysis.

| | |
|---|---|
| Injector temperature | 330°C |
| Final temperature/holding time | 330°C / 50 min |
| Duration of analysis | 78 min |

**High Performance Liquid Chromatography.** The analysis was carried out by using an HPLC-system consisting of a gradient pump, an autosampler, column thermostat and UV-detector (Agilent or Dionex).
**A) Compounds of formula VII'.** A sample (25 mg) was weighed into a 50-mL volumetric flask, and dissolved in acetonitrile. An aliquot of this solution (10 µL) was assayed by high performance liquid chromatography, using a YMC Pack Pro C18 RS column (25 cm x 4.6 mm ID, 5 µm particle) maintained at 40°C and UV detection (220 nm). Flow rate: 1.0 ml/min. Gradient solvent system (A: 20 mM KH₂PO₄ buffer, pH 3.0;
B: acetonitrile):

| time (min) | A (%) | B (%) |
|---|---|---|
| 0 | 50 | 50 |
| 20 | 5 | 95 |
| 25 | 5 | 95 |
| 25.3 | 50 | 50 |
| 30 | 50 | 50 |

**B) Compounds of formula VIII', X'.** A sample (25 mg) was weighed into a 50-mL volumetric flask, and dissolved in 25 mL acetonitrile. Water was added to 50.0 mL. An aliquot of this solution (10 µL) was assayed by high performance liquid chromatography, using a YMC Pack Pro C18 RS column (25 cm x 4.6 mm ID, 5 µm particle) maintained at 40°C and UV detection (290 nm). Flow rate:1.0 ml/min. Gradient solvent system (A: 10 mM ammonium formate buffer, pH 3.0; B: acetonitrile):

| time (min) | A (%) | B (%) |
|---|---|---|
| 0 | 90 | 10 |
| 20 | 10 | 90 |
| 22 | 10 | 90 |
| 23 | 90 | 10 |
| 30 | 90 | 10 |

**LC-MS.** A sample (5 mg) was dissolved in 10 mL acetonitrile and diluted to 25.0 mL with acetonitrile / water (1 : 1). An aliquot of this solution (5 µL) was assayed by high performance liquid chromatography, using a Purospher STAR RP18 endcapped column (12.5 cm x 2 mm ID, 5 µm particle) maintained at 25°C and MS detection in ESI positive mode. Flow rate: 0.3 ml/min. Gradient solvent system (A: water with 0.05% formic acid;
B: acetonitrile with 0.05% formic acid).

| time (min) | A (%) | B (%) |
|---|---|---|
| 0 | 80 | 20 |
| 20 | 0 | 100 |
| 25 | 0 | 100 |
| 26 | 80 | 20 |
| 35 | 80 | 20 |

**C) Compounds of formula XI'.** A sample (25 mg) was weighed into a 50-mL volumetric flask, and dissolved in 25 mL acetonitrile and 20 mL of water. Water was added to 50.0 mL. An aliquot of this solution (10 µL) was assayed by high performance liquid chromatography, using a YMC Pack Pro C18 RS column (25 cm x 4.6 mm ID, 5 µm particle) maintained at 40°C and UV detection (290 nm). Flow rate: 1.0 ml/min. Gradient solvent system (A: water with 0.05% formic acid; B: acetonitrile).

| time (min) | A (%) | B (%) |
|---|---|---|
| 0 | 90 | 10 |
| 20 | 10 | 90 |
| 22 | 10 | 90 |
| 23 | 90 | 10 |
| 30 | 90 | 10 |

**(LC-MS).** A sample (5 mg) was dissolved to 100.0 mL in a mixture of acetonitrile / water (1 : 1). An aliquot of this solution (5 µL) was assayed by high performance liquid chromatography, using a Purospher STAR RP18 endcapped column (12.5 cm x 2 mm ID, 5 µm particle) maintained at 25°C and MS detection in ESI positive mode (abbreviated as ESI(+) in the following Examples). Flow rate: 0.3 ml/min. (A: water with 0.05% formic acid; B:
acetonitrile with 0.05% formic acid).

| time (min) | A (%) | B (%) |
|---|---|---|
| 0 | 80 | 20 |
| 20 | 0 | 100 |
| 25 | 0 | 100 |
| 26 | 80 | 20 |
| 35 | 80 | 20 |

### Example 1: Preparation of compounds of formula VII'

### Benzenesulfonicacid-4-[6-methoxy-2-(4-methoxy-phenyl)-benzo[b]thiophene-3-carbonyl]-phenyl ester

### a) Preparation by using Fe powder / Fe₂(SO₄)₃ x 5 H₂O as the catalyst and toluene as the solvent:

A mixture of 68.1 g of a solution of benzenesulfonic acid 4-chlorocarbonyl-phenyl ester (19.3 g, 65 mmol, calcd.), toluene (107 mL), 6-methoxy-2-(4-methoxyphenyl)- benzothiophene (14.1 g, 52 mmol), iron (0.242 g, 4 mmol) and Fe₂(SO₄)₃ x 5 H₂O (1.898 g, 4 mmol) was heated to 106 °C to initiate the reaction. The dark coloured mixture was subsequently treated with 6-methoxy-2-(4-methoxy-phenyl)benzothiophene (126.48 g, 468 mmol) and toluene (107 mL). 612.7 g of a solution of benzenesulfonic acid 4-chlorocarbonyl-phenyl ester in toluene (173.57 g, 585 mmol, calcd.) were added slowly (5.5 h) at 100 - 111 °C with stirring. The mixture was then cooled to 98 °C and flushed with nitrogen for 11 h at 99 - 104 °C. The reaction mixture was cooled to 62 - 66 °C, diluted with toluene (637 ml), treated with sodium hydroxide (aq., 1 N, 328 mL), and SiO₂ (11.57 g) with stirring. Stirring was continued for 30 min at 62 - 66 °C and insolubles were filtered off. The filter residue was rinsed with hot toluene (40 mL). The combined biphasic filtrates were separated. The aqueous phase was disposed. The organic phase was alkalized with sodium hydroxide (aq., 1 N, 328 mL) and heated at 60 - 64 °C for approximately 40 min with vigorous stirring. The phases were separated and the aqueous phase was disposed. The organic phase was washed with water (3 x 328 mL) and the solvent was distilled off at 62 - 66 °C in vacuo to yield 301.6 g of benzenesulfonic acid-4-[6-methoxy-2-(4-methoxy-phenyl)-benzo[b]thiophene-3-carbonyl]-phenyl ester.

For an optional purification, the residue was treated with acetone (240 mL) and heated to reflux with stirring. After 10 min the clear solution was cooled to 1 - 4 °C and stirring was continued for further 60 min. The suspension was filtered and the solid washed with cold acetone (3 x 40 mL) to give the title compound as crystalline solid. Yield: 183.2 g (66.4 %, calcd. dry). **Purity**: 96.74 % (HPLC). **XRPD** the XRD diffractorgram exhibits signals at the following 2θ values:

| **2theta[°]** | **l/l₀** |
|---|---|
| 8.81 | 208.7 |
| 11.15 | 182.15 |
| 12.93 | 585.91 |
| 13.77 | 600.06 |
| 14.42 | 210.08 |
| 14.84 | 77.14 |
| 16.16 | 691.36 |
| 17.07 | 112.41 |
| 18.73 | 239.91 |
| 19.51 | 124.75 |
| 19.63 | 119.23 |
| 20.09 | 156.51 |
| 20.22 | 130.03 |
| 21.46 | 307.24 |
| 22.26 | 1000 |
| 23.11 | 513.89 |
| 23.87 | 241.67 |
| 24.29 | 68.5 |
| 24.61 | 199.52 |
| 24.88 | 209.9 |
| 25.76 | 55.71 |
| 26.03 | 125.04 |
| 26.19 | 65.87 |
| 26.74 | 193.14 |
| 27.67 | 136.14 |
| 27.79 | 173.01 |
| 28.09 | 83.15 |
| 29.11 | 67.1 |
| 30.39 | 92.73 |
| 34.17 | 81.95 |

### b) Preparation by using FeCl₃ as the catalyst and toluene as the solvent:

A mixture of benzenesulfonic acid 4-chlorocarbonyl-phenyl ester (26.7 g, 0.090 mol), toluene (135 mL), 6-methoxy-2-(4-methoxyphenyl)benzothiophene (19.5 g, 0.072 mol), and FeCl₃ (0.292 g, 0.002 mol) was stirred for 1.5 h at 23 - 24 °C and for 4.5 h at 56 - 63 °C. The mixture was treated with sodium hydroxide (aq., 1 N, 50 mL) and toluene (60 mL) with stirring. The mixture was filtered and the phases separated. The aqueos phase was discarded. The organic phase was washed with water (3 x 70 mL) and concentrated at 40 °C under reduced pressure. Methanol (75 mL) was added and the suspension was stirred for 0.5 h at 0 - 5 °C. The solid was separated; washed a mixture of toluene and methanol (1:1 v/v, .2 x 10 mL) and dried at 50 °C in vacuo. **DSC**: peak at 128.1 °C.

For optional purification, a sample (26.4 g) of the product obtained above was dissolved in hot toluene (45 mL). The turbid solution was filtered and the filter washed with toluene (3 mL). The combined filtrates were cooled to 4 °C with stirring and filtered. The solid was washed with toluene (2 x 10 mL) and dried at 50 °C under reduced pressure.

A sample (15.0 g) of the re-crystallized product was dissolved in hot acetone (35 mL). The clear solution was allowed to reach approx. 30 °C. The resulting suspension was cooled to 2-3 °C and stirring was continued for further 60 min. The solid was isolated, washed acetone (2 x 4 mL) and dried at 40 °C under reduced pressure. Yield: 41.4 % (calcd.). **Purity**: 99.86% (HPLC). DSC: peak at 134.2 °C. **¹H NMR** (300 MHz, CDCl₃, ppm): δ 7.66 (m, 3 H, arom.), 7.60 (m, 3 H, arom.), 7.46 (m, 2 H, arom.), 7.27 (s, 1H, arom.), 7.17 (d, 2 H, arom.), 6.96 (d, 1 H, arom.), 6.76 (d, 2 H, arom.), 6.68 (d, 2 H, arom.), 3.83 (s, 3 H, -OCH₃), 3.71 (s, 3 H, -OCH₃); **¹³C-{H}-NMR** (75 MHz, CDCl₃, ppm): δ 192.46 (>C=O), 160.05, 157.90, 152.51, 145.49, 140.11, 136.53, 135.21 (=C<), 134.35 (=CH-), 133.58 (=C<), 131.41, 130.67 (=CH-), 129.66 (=C<), 129.16, 128.37 (=CH-), 125.73 (=C<), 124.21, 122.11, 115.05, 113.98, 104.57 (=CH-), 55.64, 55.27 (-OCH₃). **IR** (KBr, cm⁻¹): 3068, 3006, 2961, 2907, 2836, 1641 (*v*_{C=O}), 1606 (*v*_{arom.}), 1530, 1493, 1477, 1450, 1438, 1409, 1376, 1355, 1327, 1290, 1253, 1218, 1204, 1154, 1132, 1093, 1046, 1030, 1015, 892, 863, 841, 785, 750, 730, 711, 687, 617, 581. **GC/MS** (EI, RT; 47.138 min): m/z = 530 [M]^{+·}.

### c) Preparation by using Fe / Fe₂(SO₄)₃ as the catalyst and chlorobenzene as the solvent employing 0.02 eq. of iron ions

A mixture of benzenesulfonic acid 4-chlorocarbonyl-phenyl ester (26.7 g, 0.090 mol), chlorobenzene (133 mL), 6-methoxy-2-(4-methoxyphenyl)benzothiophene (19.5 g, 0.072 mol), iron (0.0335 g, 0.0006 mol), and Fe₂(SO₄)₃ (0.263 g, 0.0013 mol) was stirred for 4 h at 56 - 63 °C. The organic phase was washed with sodium hydroxide (aq., 1 N) and water and taken to dryness at 65 °C under reduced pressure.

### Methanesulfonic acid-4-[6-methoxy-2-(4-methoxy-phenyl)-benzo[b]thiophene-3-carbonyl]-phenyl ester

A mixture of methanesulfonic acid 4-chlorocarbonyl-phenyl ester (7.5 g, 31.96 mmol), toluene (30 mL), 6-methoxy-2-(4-methoxyphenyl)benzothiophene (6.9 g, 25.5 mmol), iron (0.012 g, 0.22 mmol), and Fe₂(SO₄)₃ x 5 H₂O (0.093 g, 0.22 mmol) was heated at reflux for 9 h with stirring. The mixture was then flushed with nitrogen for 7.5 h at 108 - 110°C, cooled to 70 °C and treated with toluene (30 mL), sodium hydroxide (aq., 1 N, 16 mL), and SiO₂ (0.6 g). Stirring was continued for 50 min at 70 - 72 °C and the suspension was filtered. The filter residue was washed with hot toluene (5 mL) and the filtrates were combined. The phases were separated. The aqueous phase was disposed. The organic phase was treated with sodium hydroxide (aq., 1 N, 16 mL) and the mixture was stirred for 30 min at 75 °C. The phases were separated and the organic phase was washed with water (3 x 16 mL) at 90 °C. The organic phase was cooled to 1 °C and stirring was continued for 40 min. The solid was filtered off, washed with cold toluene (3 x 3 mL) and dried at 45 °C under reduced pressure. Yield: 5.26 g (11.2 mmol, 44.05 %).
**Purity**: 95.01 % (HPLC).

A sample (4.84 g, 10.3 mmol) of the above obtained product was suspended in acetone (55 mL) and heated to reflux. The resulting solution was cooled to 52 °C and seeded. The turbid solution was cooled to 1 3 °C and stirring was continued for 1 h. The solid was isolated, washed with cold acetone (3 x 1 mL) and dried at 45 °C under reduced pressure. Yield: 3.88 g (8.3 mmol, 80.0 %). **Purity:** 98.99 % (HPLC). **DSC:** peak at 163.08 °C.
**¹H NMR** (300 MHz, DMSO-d₆, ppm): δ7.75 (d, 2 H, arom.), 7.67 (d, 1 H, arom.), 7.58 (d, 1 H, arom.), 7.37 - 7.18 (m, 4 H, arom.), 7.06 (dd, 1 H, arom.), 6.83 (d, 2 H, arom.), 3.85 (s, 3 H, -OCH₃), 3.69 (s, 3 H, -OCH₃), 3.33 (s, 3 H, -OSO₂CH₃). **¹³C-{H}-NMR** (75 MHz, DMSO-d₆, ppm): δ 192.07 (>C=O), 159.66 (=C<), 157.47(=C<), 152.21(=C<), 144.37(=C<), 139.45(=C<), 135.89(=C<), 132.84(=C<), 131.46 (=CH-), 130.24 (=CH-), 129.18(=C<), 124.93(=C<), 123.59 (=CH-), 122.14 (=CH-), 115.18 (=CH-), 114.13 (=CH-), 105.11 (=CH-), 55.54 (-OCH₃), 55.14 (-OCH₃), 37.41 (-CH₃). **IR** (KBr, cm⁻¹): 3015, 2939, 2837, 1640 (v_{c=o}), 1606, 1527, 1493, 1473, 1440, 1362, 1298, 1250, 1222, 1172, 1148, 1127, 1047, 1028, 959, 892, 871, 862, 840, 786, 769, 717, 633, 615, 556, 527, 517. **GC/MS** (EI, RT; 39.900 min): m/z = 468 [M]^{+·}. **XRPD:** the X-ray diffractogram exhibits signals at the following 2θ values:

| **2theta[°]** | **l/l₀** |
|---|---|
| 6.21 | 71.16 |
| 11.44 | 144.14 |
| 12.25 | 62.4 |
| 12.97 | 99.06 |
| 14.51 | 86.7 |
| 15.31 | 59.98 |
| 16.31 | 57.5 |
| 17.86 | 222.06 |
| 18.77 | 479.76 |
| 18.93 | 408.11 |
| 19.07 | 753.68 |
| 19.23 | 122.2 |
| 19.39 | 145.69 |
| 19.77 | 1000 |
| 21.25 | 70.77 |
| 22.21 | 108.58 |
| 23 | 148.5 |
| 23.84 | 140.58 |
| 24.64 | 262.97 |
| 25.44 | 193.37 |
| 25.57 | 130.08 |
| 26.11 | 251.36 |
| 28.26 | 377.94 |
| 29.76 | 65.39 |
| 29.92 | 136.45 |
| 30.3 | 112.15 |
| 39.06 | 50.3 |

### Toluenesulfonic acid-4-[6-methoxy-2-(4-methoxy-phenyl)-benzo[b]thiophene-3-carbonyl]-phenyl ester

A mixture of toluenesulfonic acid 4-chlorocarbonyl-phenyl ester (10.0 g, 32.18 mmol), toluene (40 mL), 6-methoxy-2-(4-methoxyphenyl)benzothiophene (6.96 g, 25.7 mmol), iron (0.012 g, 0.22 mmol), and Fe₂(SO₄)₃ x 5 H₂O (0.094 g, 0.22 mmol) was heated at reflux for 10.5 h with stirring. The mixture was cooled to approx. 70 °C, diluted with toluene (40 mL), treated with sodium hydroxide (aq., 1 N, 16 mL) and SiO₂ (0.6 g) and stirring was continued for 30 min at 65 - 68 °C. The mixture was filtered and the residue washed with hot toluene (5 mL). The filtrates were combined and the phases separated. The aqueous phase was disposed. The organic phase was treated with sodium hydroxide (aq., 1 N, 16 mL) and stirring was continued for 15 min at 60 - 68 °C. The phases were separated and the organic phase was washed with water (3 x 16 mL) at ambient temperature, concentrated at 45 °C under reduced pressure, and cooled to 1 °C with stirring. After 60 min the resulting solid was isolated, washed with cold toluene (2 x 1 mL) and dried at 45 °C under reduced pressure. Yield 8.17 g (15.0 mmol, 58.4 %). Purity: 84.37 % (HPLC).

A sample (7.56 g,13.88 mmol) of the material obtained above was dissolved in toluene (19 mL) at approx. 100 °C. The solution was cooled to 5 °C with seeding and stirring was continued for 1.5 h. The resulting solid was filtered off, washed with cold toluene (2 x 2 mL), and dried at 50 °C under reduced pressure. Yield 4.72 g (8.7 mmol, 62.4 %). The re-crystallized material (4.55 g, 8.35 mmol) was suspended in acetone (13 mL) and heated to reflux. The resulting solution was cooled to 1 °C with seeding and stirring was continued for 45 min. The solid was isolated, washed with cold acetone (2 x 0.75 mL) and dried at 65 °C to give the title compound as crystalline solid. Yield: 3.49 g (6.4 mmol, 76.7 %). **Purity:** 92.33 % (HPLC). **Mp.:** 132.9 - 134.0 °C. **DSC:** peak at 133.14 °C. ¹H **NMR** (300 MHz, DMSO-d₆, ppm): δ 7.70 - 7.50 (m, 6 H , arom.), 7.44 (m, 2 H, arom.), 7.22 (d, 2 H, arom.), 7.04 (dd, 1 H, arom.), 6.93 (d, 2 H, arom.), 6.85 (d, 2 H9, arom.), 3.84 (s, 3 H, -OCH₃ ), 3.72 (s, 3 H, -OCH₃) 2.41 (s, 3H, -CH₃)- **¹³C-{H}-NMR** (75 MHz, DMSO-d₆, ppm): δ 191.87 (>C=O), 159.68 (=C<), 157.46 (=C<), 152.00 (=C<), 145.98 (=C<), 144.46 (=C<), 139.47 (=C<), 135.90 (=C<), 132.82 (=C<), 131.28 (=CH-), 131.05 (=C<), 130.23 (=CH-), 130.11 (=CH-), 128.98 (=C<), 128.02 (=CH-), 124.90 (=C<), 123.54 (=CH-), 122.13 (=CH-), 115.14 (=CH-), 114.14 (=CH-), 105.08 (=CH-), 55.52 (-OCH₃), 55.18 (-OCH₃), 21.9 (-CH₃). **IR** (KBr, cm⁻¹): 2947, 2844, 1649 (v_{C=O}), 1598, 1527, 1497, 1476, 1442, 1376, 1356, 1294, 1251, 1215, 1201, 1177, 1154, 1092, 1045, 1022, 893, 862, 838, 739, 726, 668, 617, 573, 553. **GC/MS** (EI, RT; 71.400 min): m/z = 544 [M]^{+·}. **XRPD**:the X-ray diffractogram exhibits signals at the following 2θ values:

| **2theta[°]** | **l/l₀** |
|---|---|
| 6.04 | 247.1 |
| 7.54 | 202.01 |
| 10.84 | 177.43 |
| 12.09 | 595.56 |
| 13.05 | 240.93 |
| 14.38 | 118.19 |
| 14.81 | 125.76 |
| 16.05 | 487.7 |
| 17.59 | 1000 |
| 17.86 | 333.83 |
| 18.01 | 176.09 |
| 18.97 | 486.46 |
| 19.18 | 83.42 |
| 19.41 | 86.52 |
| 20.4 | 267.28 |
| 20.53 | 299.19 |
| 21.05 | 522.54 |
| 21.3 | 200.27 |
| 22.57 | 289.73 |
| 23.03 | 623.01 |
| 24.17 | 82.34 |
| 24.37 | 128.02 |
| 25.3 | 148.52 |
| 26.08 | 87.7 |
| 26.44 | 584.05 |
| 26.85 | 262.88 |
| 27.97 | 92.12 |
| 29.07 | 95.11 |
| 31.1 | 96.61 |
| 32.46 | 85.66 |

### Example 2: Preparation of compound of formula VIII'

### (4-Hydroxy-phenyl)-[6-methoxy-2-( 4-methoxy-phenyl)-benzo[b]thiophen-3-yl]-methanone

### a) Preparation by using moist benzenesulfonic acid-4-[6-methoxy-2-(4-methoxy-phenyl)-benzo[b]thiophene-3-carbonyl]-phenyl ester, TBAB as the catalyst, NaOH as the base and toluene/water as the solvent:

A 2 L glass flask was charged with benzenesulfonic acid-4-[6-methoxy-2-(4-methoxy-phenyl)-benzo[b]thiophene-3-carbonyl]-phenyl ester (182.5 g, 344 mmol, calcd. dry), prepared as described under example 1 item a, TBAB (2.2 g, 7 mmol), and toluene (365 mL). The contents were heated to 106 -108 °C and 22 mL of solvents were distilled off. The mixture was cooled to 80 °C and treated with water (160 mL) and aq. sodium hydroxide (92 mL, 1720 mmol). The biphasic mixture was heated to reflux with stirring. After 4 h water (365 mL) was added and stirring was continued for 20 min at 88 °C. The phases were separated and the organic phase was disposed. The aqueous phase was vigorously washed with toluene (290 mL) at approx. 90 °C, cooled to 12 °C, and treated with methylene chloride (670 mL) and hydrochloric acid (aq., 175 mL, 1800 mmol) in sequence. Stirring was continued for 25 min at 12 - 20 °C until a pH of 1 was observed. The phases were separated and the organic phase was washed with water (3 x 165 mL) to yield a solution of (4-hydroxy-phenyl)-[6-methoxy-2-(4-methoxy-phenyl)-benzo[b]thiophen-3-yl]-methanone in methylene chloride (1002 g). **Purity:** 97.62 % (HPLC).

### b) Preparation by using TBAB as the catalyst, NaOH as the base and toluene/water as the solvent:

A flask was charged with benzenesulfonic acid-4-[6-methoxy-2-(4-methoxy-phenyl)-benzo[b]thiophene-3-carbonyl]-phenyl ester (27.91 g, 0.053 mol), TBAB (0.34 g, 0.0011 mol), and toluene (170 mL). Aq. sodium hydroxide (cc., 16.9 mL, 0.318 mol) and water (25 mL) were added at ambient temperature and the mixture was heated to reflux with stirring. After 6 h the mixture was treated with water (68 mL). The aqueous phase was separated and washed with toluene (45 mL) at 80 - 86 °C. The aqueous phase was subsequently treated with methylene chloride (100 mL) and aq. hydrochloric acid (cc., 32 mL, 0.331 mol) at room temperature with stirring. The organic phase was separated, washed with water (4 x 25 mL), and evaporated to dryness. A mixture of MTBE (12 mL), toluene (110 mL), and activated charcoal (1.0 g) was added after which the suspension was heated to reflux. The suspension was filtered and the residue washed with a hot mixture of MTBE and toluene (1:9 v:v, 10 mL). The combined filtrates were cooled to 2°C with stirring. The resulting precipitate was isolated; washed with a mixture of MTBE and toluene (1:9 v/v, 2 x 5 mL) and dried at 40 °C under reduced pressure. The product was dissolved in methanol (100 mL) and evaporated to dryness. This procedure was repeated twice. The residue was dried under reduced pressure to yield the title compound as an amorphous solid. Yield: 80.9 %. **Purity:** 99.21% (HPLC). **Mp.:** 79.8 - 83.2 °C. DSC: first peak at 69.8 °C, second peak at 368.6 °C. ¹H **NMR** (300 MHz, DMSO-d₆, ppm): δ 10.47 (b, 1 H, -OH), 7.62 (m, 3 H, arom.), 7.33 (m, 3 H, arom.), 6.98 (m, 1 H, arom.), 6.88 (d, 2 H, arom.), 6.74 (d, 2 H, arom.), 3.83 (s, 3 H, -OCH₃), 3.71 (s, 3 H, -OCH₃); **¹³C-{H}-NMR** (75 MHz, DMSO-d₆, ppm): δ 192.03 (>C=O), 162.61, 159.33, 157.24, 140.20, 139.24, 133.21 (=C<), 132.00 (=CH-), 130.33 (=C<), 129.50 (=CH-), 128.19, 125.21 (=C<), 123.21, 115.35, 114.81, 114.21, 105.00 (=CH-), 55.41, 55.04 **(-OCH₃). IR** (KBr, cm⁻¹): 3277, 2935, 2834, 1633 (v_{C=O}), 1603 (v_{arom.}), 1574, 1535, 1499, 1474, 1438, 1349, 1254, 1178, 1162, 1076, 1046, 1030, 893, 829, 783, 616. **LC/MS** (ESI(+), RT; 17.02 min): m/z = 391 [MH]⁺.

### c) Preparation by using TBAB as the catalyst, KOH and toluene/water as the solvent:

(4-Hydroxy-phenyl)-[6-methoxy-2-(4-methoxy-phenyl)-benzo[b]thiophen-3-yl]-methanone was also obtained in a similar manner as described under example 2 item b, employing aqueous potassium hydroxide solution (6.0 eq.) as base. Purity: 97.13 % (HPLC).

### Example 3: Preparation of sodium and potassium salts of compound of formula VIII'

### Sodium 4-[6-methoxy-2-(4-methoxy-phenyl)-benzo[b]thiophene-3-carbonyl]-phenolate

### a) Using sodium methylate as the base and methanol as the solvent:

(4-Hydroxy-phenyl)-[6-methoxy-2-(4-methoxy-phenyl)-benzo[b]thiophen-3-yl]-methanone (14.61 g, 0.024 mol), prepared as described under example 2 item b, was dissolved in methanol (14.5 mL) at ambient temperature. MTBE (44 mL) and a solution of sodium methylate in methanol (7.8 mL, 1.1 eq.) were added to the clear solution. After addition of MTBE (27 mL), the mixture was cooled and stirred for 4 h at -16 to -17 °C. The amorphous solid was separated, washed with a mixture of methanol and MTBE (40:10 v/v, 2 x 5 mL) and dried at 45 °C under reduced pressure. **Purity:** 99.28 % (HPLC). **DSC:** peak at 96.8 °C. ¹H NMR (300 MHz, DMSO-d₆, ppm): δ 7.58 (d, 1 H, arom.), 7.48 - 7.19 (m, 5 H, arom.) 6.95 (dd, 1 H, arom.) 6.90 (d, 2 H, arom.), 6.19 (b, 2 H, arom.), 3.83 (s, 3 H, -OCH₃), 3.72 (s, 3 H, -OCH₃). **IR** (KBr, cm⁻¹): 2938, 2835, 1608 (*v*_{C=O}), 1569, 1498, 1473, 1438, 1344, 1280, 1258, 1215, 1180, 1157, 1075, 1045, 1025, 894, 830, 785, 737, 644, 618, 595, 554. **LC/MS** (ESI(+), RT; 19.88): m/z = 390.9 [MH-Na]⁺.

### b) Sodium 4-[6-methoxy-2-(4-methoxy-phenyl)-benzo[b]thiophene-3-carbonyl]-phenolate using 2-piperidin-1-yl-ethanolate (formed in situ) as the base and toluene as the solvent:

A flask was charged with a solution of toluene (5 mL), sodium (0.64 g, 0.0277 mol), and 2-piperidin-1-yl-ethanol (4.6 mL, 0.0342 mol) and benzenesulfonic acid-4-[6-methoxy-2-(4-methoxy-phenyl)-benzo[b]thiophene-3-carbonyl]-phenyl ester (7.0 g, 0.0132 mol) in toluene (55 mL) was added within 20 min at 18 - 23 °C. After stirring for 1 h at ambient temperature, the mixture was heated at 47-50 °C for 0.5 h with stirring. The resulting solid was separated by filtration, washed with toluene (3 x 10 mL) and dried to give the title compound as its sodium salt.

### Potassium 4-[6-methoxy-2-(4-methoxy-phenyl)-benzo[b]thiophene-3-carbonyl]-phenolate

### a) Potassium 4-[6-methoxy-2-(4-methoxy-phenyl)-benzo[b]thiophene-3-carbonyl]-phenolate by using 2-piperidin-1-yl-ethanolate (formed in situ) as the base and toluene as the solvent:

A corresponding potassium salt was obtained in a similar manner as described under example 3 item b, employing potassium for forming 2-piperidin-1-yl-ethanolate as base.

### b) Potassium 4-[6-methoxy-2-(4-methoxy-phenyl)-benzo[b]thiophene-3-carbonyl]-phenolate using K₂CO₃ as the base:

A mixture of benzenesulfonic acid-4-[6-methoxy-2-(4-methoxy-phenyl)-benzo[b]thiophene-3-carbonyl]-phenyl ester (3.0 g, 0.0056 mol), 2-piperidin-1-yl-ethanol (9 mL, 67 mmol), and K₂CO₃ (1.88 g, 2.4 eq.) was stirred for 6.5 h at 80 - 83 °C. The mixture was cooled and extracted with a mixture of 45 mL of toluene (45 mL) and water (25 mL). The organic phase was extracted with water (2 x 25 mL) and evaporated to dryness. Yield: 85 %.

### Examples 4 and 5: Preparations of compounds of formula X'

### Example 4

### 1-(2-{4-[6-Methoxy-2-(4-methoxy-pheny)-benzo[b]thiophene-3-carbonyl]-phenoxy}-ethyl)-piperidine

### a) Preparation by using NaOH as the base and water/methylene chloride as the solvent:

A solution (830.5 g) of (4-hydroxy-phenyl)-[6-methoxy-2-(4-methoxy-phenyl)-benzo[b]thiophen-3-yl]-methanone (118.4 g, 0.303 mol, calcd. dry) in methylene chloride, prepared as described under example 2 item a, was treated with water (45 mL), 1-(2-chloro-ethyl)-piperidinium chloride (58.57 g, 0.318 mol), and methylene chloride (175 mL). Aq. sodium hydroxide (cc., 40.6 mL, 0.758 mol) was added slowly (15 min) at 16 - 20 °C with stirring. The mixture was heated to reflux and stirring was continued for 6.5 h. The contents were cooled to 24 °C and water (765 mL) was added with stirring. The phases were separated. The organic phase was washed with water (3 x 260 mL) and concentrated at 40 - 42 °C. The solution was diluted with methanol (510 mL) and concentrated at approx. 60 °C to give 478 g of a solution of [6-Methoky-2-(4-methoxy-phenyl)-benzo[b]thiophen-3-yi]-[4-(2-piperidin-1-yl-ethoxy)-phenyl]-methanone in methanol. Yield: quantitative. **Purity**: 97.0 % (HPLC).

### b) Preparation by using polyethylene glycol 600 as the catalyst, K₂CO₃ as the base and toluene/MTBE as the solvent:

A mixture of (4-hydroxy-phenyl)-[6-methoxy-2-(4-methoxy-phenyl)-benzo[b]thio-phen-3-yl]-methanone (3.9 g, 0.010 mol), toluene (25 mL), MTBE (5 mL), 1-(2-chloro-ethyl)-piperidine hydrochloride (2.03 g, 0.011 mol), K₂CO₃ (4.42 g, 0.032 mol), and PEG 600 (0.6 g, 0.001 mol) was heated at 62 - 68 °C for 16 h with stirring. The mixture was cooled and washed with water (5 x 20 mL) at ambient temperature. The organic phase was dried over Na₂SO₄ and evaporated to dryness. Yield: 88.0 %. **Purity**: 91.0 % (HPLC).

### c) Synthesis from 1-(2-{4-[6-Methoxy-2-(4-methoxy-phenyl)-benzo[b]thiophene-3-carbonyl]-phenoxy}-ethyl)-piperidinium chloride

A flask was charged with 1-(2-{4-[6-Methoxy-2-(4-methoxy-phenyl)-benzo-[b]thiophene-3-carbonyl]-phenoxy}-ethyl)-piperidinium chloride (145.0 g, 269.5 mmol), methylene chloride (750 mL), and water (250 mL). Aq. sodium hydroxide (18.86 N, 17.1 mL) was added dropwise at 18 °C with stirring. Stirring was continued for 10 min and the phases were separated. The organic phase was washed with water (2 x 250 mL) to give a solution (795 mL) of the title compound in methylene chloride. A sample (70 mL) was evaporated to dryness at 45°C under reduced pressure to give 1-(2-{4-[6-Methoxy-2-(4-methoxy-phenyl)-benzo[b]thiophene-3-carbonyl]-phenoxy}-ethyl)-piperidin. Yield: 11.58 g (23.08 mmol, 97.3 %, calcd.).
**Purity**: 97.42 (HPLC). **¹H NMR** (300 MHz, DMSO-d₆, ppm): δ 7.67 (d, 2 H, arom.), 7.64 (d, 1 H, arom.), 7.37 - 7.26 (m, 3 H, arom.), 6.98 (dd, 1 H, arom.), 6.94 (m, 4 H, arom.), 4.05 (t, 2 H, -OCH₂CH₂NR₂), 3.83 (s, 3 H, -OCH₃), 3.70 (s, 3 H, -OCH₃), 2.58 (-OCH₂CH₂NR₂), 2.36 (bt, 4 H, piperidine), 1.50 -1.27 (m, 6 H, piperidine). **¹³C-{H}-NMR** (75 MHz, DMSO-d₆, ppm): δ 192.26 (R₂CO), 162.88 (=C<), 159.45 (=C<), 157.35 (=C<), 140.7 (=C<), 139.33 (=C<), 133.18 (=C<), 131.73 (=CH-), 130.16 (=C<), 129.6 (=CH-), 129.52 (=C<), 125.17(=C<), 123.24 (=CH-), 114.93 (=CH-), 114.52 (=CH-), 114.31 (=CH-), 105.12 (=CH-), 65.98 (-CH₂-), 57.01 (-CH₂-), 55.5 (-OCH₃), 55.12 (-OCH₃), 54.25 (-CH₂-), 25.48 (-CH₂-), 23.81 (-CH₂-).
**IR** (KBr, cm⁻¹): 2933, 1647 (ν_{C=O}), 1598, 1572, 1535, 1500, 1475, 1294, 1253, 1164, 1031, 892, 828, 782, 620. **LC/MS** (ESI(+), RT; 14.17 min): m/z = 501.9 [MH]⁺.

### 1-(2-{4-[6-Methoxy-2-(4-methoxy-phenyl)-benzo[b]thiophene-3-carbonyl]-phenoxy}-ethyl)-piperidinium chloride

### d) Preparation by using TBAB as the catalyst, NaOH as the base and water/methylene chloride as the solvent:

A flask was charged with (4-hydroxy-phenyl)-[6-methoxy-2-(4-methoxy-phenyl)-benzo[b]thiophen-3-yl]-methanone (17.6 g, 0.045 mol), prepared according to example 2 item c, and methylene chloride (140 mL). The stirred mixture was treated with aq. sodium hydroxide (cc., 5.9 mL, 0.113 mol), water (6 mL), TBAB (0.73 g, 0.0023 mol), and 1-(2-chloro-ethyl)-piperidine hydrochloride (9.13 g, 0.050 mol) at ambient temperature. Stirring was continued for 16 h and the phases were separated. The organic phase was washed with aq. hydrochloric acid (1 N, 3 x 50 mL), water (55 mL), aq. sodium hydroxide (2 N, 55 mL), and with water (5 x 50 mL) in sequence. The organic phase was dried over Na₂SO₄ and evaporated to dryness. The residue (21.8 g) was dissolved in methanol (65 mL) and aq. hydrochloric acid (cc., 3.8 mL, 0.046 mol) was added at 51 °C. The suspension was cooled to 2 °C and stirring was continued for 0.75 h. The solid was isolated, washed with methanol (2 x 7.5 mL) and dried at 70 °C. Yield: 18.36 g (78.4 %).
**Purity**: 98.64 % (HPLC).

A sample (17.1 g, 31.78 mmol) of the material obtained above was dissolved in hot methanol (128 mL) and the solution was filtered. The clear filtrate was cooled to 1 °C with stirring. The resulting solid was isolated, washed with methanol (3 x 4.5 mL), and dried at 40 °C under reduced pressure. Yield: 14.61 g (85.4 %). **Purity**: 99.03 % (HPLC).
**Mp.**: 217.8 °C; **DSC**: first peak at 220.7 °C, second peak at 314 °C. **¹H NMR** (300 MHz, DMSO-d₆, ppm): δ 10.55 (b, 1 H, -NH), 7.71 (m, 3 H, arom.), 7.32 (m, 3 H, arom.), 7.00 (d, 3 H, arom.), 6.90 (d, 2 H, arom.), 4.44 (t, 2 H, -OCH₂CH₂NH-), 3.84 (s, 3 H, -OCH₃), 3.72 (s, 3 H, -OCH₃), 3.43 (m, 4 H, -NHCH₂CH₂-), 2.95 (b, 2 H, -OCH₂CH₂NH-), 1.77 (m, 4 H, -CH₂CH₂CH₂NR₂), 1.66 - 1.36 (m, 2H, -CH₂CH₂CH₂-); **¹³C-{H}-NMR** (75 MHz, DMSO-d₆, ppm) δ 192.22 (>C=O), 161.66 (=C<), 159.37 (=C<), 157.26 (=C<), 140.69 (=C<), 139.23 (=C<), 132.98 (=C<), 131.64 (=CH-), 130.11 (=C<), 129.94 (=C<), 129.50 (=CH-), 125.02 (=C<), 123.06 (=CH-), 114.87 (=CH-), 114.61 (=CH-), 114.27 (=CH-), 105.09 (=CH-), 62.40 (-CH₂-), 55.43 (-OCH₃), 55.08 (-OCH₃), 54.30 (-CH₂-), 52.41 (-CH₂-), 22.08 (-CH₂-), 20.95 (-CH₂-). **IR** (KBr, cm⁻¹): 3413, 2941, 2453, 1643 (ν_{C=O}), 1599 (νₐᵣₒₘ,), 1574, 1535, 1498, 1475, 1438, 1353, 1307, 1295, 1251, 1179, 1043, 1026, 949, 893, 830, 783, 645, 621. **LC/MS** (ESI(+), RT; 11.90 min): m/z = 502.1 [MH]⁺. **XRPD:** the XRD diffractogram exhibits signals at the following 2θ values:

| **2theta[°]** | **l/l₀** |
|---|---|
| 6.41 | 84.31 |
| 11.41 | 259.94 |
| 12.56 | 215.27 |
| 13.07 | 303.03 |
| 14.82 | 198.13 |
| 15.45 | 144.45 |
| 15.86 | 109.53 |
| 16.74 | 421.69 |
| 17.1 | 119.28 |
| 18.33 | 90.15 |
| 18.95 | 197.08 |
| 19.27 | 1000 |
| 19.39 | 321.4 |
| 19.79 | 66.13 |
| 20.09 | 233.2 |
| 21.56 | 278.71 |
| 22.32 | 118.66 |
| 22.92 | 114.72 |
| 23.42 | 995.98 |
| 24 | 247.55 |
| 24.63 | 780.62 |
| 24.77 | 96.24 |
| 25.53 | 236.05 |
| 25.89 | 136.45 |
| 26.05 | 320.65 |
| 26.83 | 121.67 |
| 29.35 | 147.82 |
| 29.49 | 128.56 |
| 30.45 | 67.32 |
| 42.03 | 66.09 |

### e) Preparation by using NaOH as the base and water/methylene chloride as the solvent:

A flask was charged with a solution (735 g) of (4-hydroxy-phenyl)-[6-methoxy-2-(4-methoxyphenyl)-benzo[b]thiophen-3-yl]-methanone (96.0 g, 0.246 mol, calcd. dry) in methylene chloride, prepared as described under example 2 item a, water (35 mL), 1-(2-chloro-ethyl)-piperidine hydrochloride (49.82 g, 0.271 mol), and methylene chloride (96 mL). Aq. sodium hydroxide (cc., 32.9 mL, 0.622 mol) was added slowly (20 min) at 15 -18 °C with stirring. The mixture was allowed to reach room temperature and stirring was continued for a further 60 min after which the reactor contents were heated at reflux for approx. 2.25 h. The resulting clear solution was allowed to reach room and treated with water (620 mL). Stirring was continued for a further 10 min and the phases were separated. The organic phase was washed with water (4 x 210 mL), concentrated at max. 48 °C and dissolved in methanol (385 mL). The resulting clear solution was concentrated at normal pressure and aq. hydrochloric acid (cc., 21.5 mL.) was added at approx. 50 °C with stirring. The solution was seeded with appropriate material. Stirring was continued for approx. 45 min and the resulting suspension was cooled to approx. -10 °C. Stirring was continued for a further 60 min at -6 to -9 °C after which the precipitate was isolated, washed with cold methanol (3 x 30 mL) and dried under reduced pressure (5 h, 50 °C) to give the title compound.
Yield: 99.19 g (75.0 %). **Purity**: 97.16 % (HPLC).

For an optional purification, a sample (98.3 g, 182.7 mmol) was suspended in methanol (675 mL) and heated to reflux. The resulting clear solution was seeded with appropriate material with stirring. The resulting suspension was cooled to 2 °C and stirring was continued for 1.5 h. The solid was isolated, washed with cold methanol (2 x 30 mL) and dried at 50 °C under reduced pressure. Yield: 85.7 g (159.3 mmol, 87.2 %).
**Purity**: 99.33 % (HPLC).

### f) Preparation from 1-(2-{4-[6-Methoxy-2-(4-methoxy-phenyl)-benzo[b]thiophene-3-carbonyl]-phenoxy}-ethyl)-piperidinium bromide as the starting material

1-(2-{4-[6-Methoxy-2-(4-methoxy-phenyl)-benzo[b]thiophene-3-carbonyl]-phenoxy}-ethyl)-piperidinium bromide (122.3 g, 209.9 mmol), prepared according to example 5 item c was added to mixture of methylene chloride (630 mL) and water (210 mL) with stirring. Aq. sodium hydroxide (18.86 N, 14.5 mL) was added over a period of 5 min at 17 °C and stirring was continued for 10 min. The phases were separated and the organic phase was washed with water (2 x 210 mL). Aqueous phases were disposed and the organic phase was concentrated at 45 °C under reduced pressure. Methanol (700 mL) was added to the residue and the mixture was heated to 50 °C. Hydrochloric acid (aq., 12 N, 21 mL, 252 mmol) was added dropwise to reach a pH of 1.5. The clear solution was seeded with appropriate material and, after crystallization was observed, the suspension was cooled to approx. -10 °C and stirring was continued for 2.5 h. The solid was isolated, washed with cold methanol (3 x 30.mL) and dried at 50 °C under reduced pressure to give the title compound.
Yield: 105.76 g (196.5 mmol, 93.6 %). **Purity**: 99.69 % (HPLC).

### Example 5

### 1-(2-{4-[6-Methoxy-2-(4-methoxy-phenyl)-benzo[b]thiophene-3-carbonyl]-phenoxy}-ethyl)-piperidinium bromide

### a) Preparation by using methanol as the solvent:

A solution of [6-Methoxy-2-(4-methoxy-phenyl)-benzo[b]thiophen-3-yl]-[4-(2-piperidin-1-yl-ethoxy)-phenyl]-methanone (75.0 g, 0.150 mol) in methanol (195 mL), prepared as described under example 4 item a, was heated to 50 - 58 °C and aq. hydrobromic acid (47 %, 25.9 g, 0.150 mol) was added dropwise to adjust a pH of 1. The resulting suspension was cooled to - 6 °C and stirring was continued for 0.75 h. The crystalline solid was separated, washed with cold methanol (3 x 20 mL) and dried at 50 °C under reduced pressure. Yield: 79.01 g (135.4 mmol, 89.5 %). **Purity**: 98.34 % (HPLC). **DSC**: peak at 232 °C; peak at 330 °C. **IR** (KBr, cm⁻¹): 2940, 2838, 2627, 2517, 1642 (ν_{C=O}), 1598, 1573, 1535, 1498, 1475, 1455, 1435, 1354, 1307, 1294, 1251, 1217, 1178, 1128, 1083, 1043, 1026, 1007, 970, 947, 893, 829, 783, 765, 644, 620, 512.

For an optional purification, a sample (77.4 g, 132.6 mmol) of the product as obtained above was dissolved in a mixture of 1-propanol and water (75:25 v/v, 500 mL) at reflux temperature. The solution was allowed to cool to 70 °C, seeded with appropriate material, and cooled to approximately 5 °C. Stirring was continued for a further 60 min and the solid was separated. The crystalline material was washed with a cold mixture of 1-propanol and water (75:25 v/v, 2 x 25 mL) and dried under reduced pressure (6 h, 50 °C). Yield: 70.79 g (121.3 mmol, 91.5 %); **Purity**: 99.18 % (HPLC). **Mp**.: 230.8 °C; **DSC**: first peak at 233.1 °C, second peak at 330.6 °C. **¹H NMR** (300 MHz, DMSO-d₆, ppm): δ 9.58 (bs, 1 H, - NH), 7.73 (d, 2 H, arom.), 7.66 (dd, 1 H, arom.), 7.32 (m, 3 H, arom.), 7.00 (m, 3 H, arom.), 6.89 (d, 2 H, arom.), 4.42 (t, 2 H, -OCH₂CH₂NH-), 3.84 (s, 3 H, -OCH₃), 3.72 (s, 3 H, -OCH₃), 3.50 (b, 4 H, -OCH₂CH₂NH-), 3.02 (m, 2 H, piperidine), 1.96 - 1.17 (m, 6 H, piperidine). **¹³C-{H}-NMR** (75 MHz, DMSO-d₆, ppm): δ 192.36 (>C=O), 161.78 (=C<), 159.46 (=C<), 157.36 (=C<), 140.81 (=C<), 139.36 (=C<), 133.10 (=C<), 131.76 (=CH-), 130.20 (=C<), 130.01 (=C<), 129.62 (=CH-), 125.13 (=C<), 123.17 (=CH-), 115.01 (=CH-), 114.76 (=CH-), 114.38 (=CH-), 105.18 (=CH-), 62.42 (-CH₂-), 55.55 (-OCH₃), 55.21 (-OCH₃), 54.56 (-CH₂-), 52.63 (-CH₂-), 22.27 (-CH₂-), 20.99 (-CH₂-). **IR** (KBr, cm⁻¹): 2940, 2628, 2517, 1642 (ν_{C=O}), 1598, 1573, 1535, 1498, 1475, 1454, 1435, 1354, 1307, 1294, 1251, 1217, 1173, 1127, 1043, 1026; 1007, 970, 946, 893, 829, 783, 765, 644, 620, 542, 512. **LC/MS** (ESI(+), RT; 13.99 min): m/z = 502.1 [MH]⁺. **XRPD**: the X-ray diffractogram exhibits signals at the following 2θ values:

| **2theta[°]** | **l/l₀** |
|---|---|
| 7.41 | 236.8 |
| 11.38 | 97.77 |
| 13.05 | 432.67 |
| 14.57 | 130.76 |
| 15.28 | 130.66 |
| 16.64 | 196.79 |
| 17.1 | 104.55 |
| 19.07 | 328.62 |
| 19.22 | 370.8 |
| 19.56 | 103.49 |
| 19.71 | 111.49 |
| 20.04 | 158.16 |
| 21.45 | 455.47 |
| 22.35 | 129.8 |
| 22.84 | 119.53 |
| 23.31 | 492.39 |
| 23.81 | 127.93 |
| 24.34 | 1000 |
| 25.45 | 145.39 |
| 25.81 | 606.94 |
| 25.96 | 126.15 |
| 26.12 | 101.11 |
| 26.43 | 89.73 |
| 26.62 | 213.81 |
| 27.3 | 163.65 |
| 29.3 | 120.27 |
| 30.33 | 124.47 |
| 33.65 | 100.22 |
| 33.95 | 103.18 |
| 41.36 | 102.63 |

### b) Preparation by using 2-propanol as the solvent:

1-(2-(4-[6-Methoxy-2-(4-methoxy-phenyl)-benzo[b]thiophene-3-carbonyl]-phenoxy)-ethyl)-piperidinium bromide was also obtained in a similar manner as described under example 5 item a, employing 2-propanol as solvent. Yield: 5.81 g (9.96 mmol, 83.4 %). **Purity**: 96.0 %
(HPLC). **Mp**..: 225.8 °C; **DSC**: first peak at 230.5 °C, second peak at 328.7 °C. **IR** (KBr, cm⁻¹): 2941, 2838, 2628, 2517, 1642 (ν_{C=O}), 1598, 1573, 1535, 1498, 1475, 1455, 1435, 1354, 1307, 1294, 1251, 1217, 1173, 1128, 1083, 1043, 1026, 1007, 970, 947, 893, 829, 783, 644, 621, 512. **XRPD**: XRD data correspond to that given for Example 5 a).

### c) Preparation by using n-propanol/water as the solvent:

A solution of [6-Methoxy-2-(4-methoxy-phenyl)-benzo[b]thiophen-3-yl]-[4-(2-piperidin-1-yl-ethoxy)-phenyl]-methanone (189.09 g, 376.9 mmol) in a mixture of n-propanol and water (75:25 v:v, 570 mL) prepared as described under example 4 item a, was heated to 60 - 70 °C and aq. hydrobromic acid (47 %, 42 mL, 377 mmol) was added slowly (30 min) to adjust a pH of 1.5 - 2. The resulting suspension was cooled to 2 - 5 °C and stirring was continued for 1.5 h. The solid was isolated and washed three times with a cold mixture of n-propanol/water (75:25 v:v, 3 x 40 mL). Yield: 227.2 g (172.8 g, 296.6 mmol, 86.8 %, calcd. dry). **Purity**: 98.18 % (HPLC).

### d) Preparation from 1-(2-{4-[6-Methoxy-2-(4-methoxy-phenyl)-benzo[b]thiophene-3-carbonyl]-phenoxy}-ethyl)-piperidinium chloride as the starting material

1-(2-{4-[6-Methoxy-2-(4-methoxy-phenyl)-benzo[b]thiophene-3-carbonyl]-phenoxy}-ethyl)-piperidinium chloride (145.0 g, 269.5 mmol), prepared according example 4 item e was added to a mixture of methylene chloride (750 mL) and water (250 mL) with stirring. Aq. sodium hydroxide (18.86 N, 17.1 mL) was added dropwise over a period of 5 min at 18 °C and stirring was continued for 10 min. The phases were separated and the organic phase was washed water (2 x 250 mL). Yield: 795 mL of a solution of 1-(2-{4-[6-Methoxy-2-(4-methoxy-phenyl)-benzo[b]thiophene-3-carbonyl]-phenoxy}-ethyl)-piperidine in methylene chloride. A samples (655 mL) was evaporated at 45 °C under reduced pressure and treated with a mixture of 1-propanol (525 mL) and water (160 mL). The mixture was heated to 70 °C and aq. hydrobromic acid (48%, 29 mL, 258 mmol) was added dropwise over a period of 8 min to adjust a pH of 1.5. The solution was allowed to cool to 77 °C and seeded with appropriate material. After crystallization was observed the suspension was cooled to 0 - 5 °C and stirring was continued for 2 h. The solid was isolated, washed with a cold mixture of 1-propanol and water (75:25 v/v, 3 x 40 mL) and dried at 50 °C under reduced pressure. Yield: 121.24 g (208.1 mmol, 77.2 %). **Purity**: 99.3 % (HPLC). **IR** (KBr, cm⁻¹): 2940, 2627, 2517, 1642 (ν_{C=O}), 1598, 1573, 1534, 1498, 1475, 1454, 1435, 1354, 1307, 1294, 1251, 1217, 1173, 1127, 1083, 1043, 1026, 1007, 969, 946, 893, 829, 783, 764, 644, 620, 533, 512.

### Example 6 to 10: Preparation of compound of formula Xl'

### Example 6

### Crystalline [6-Hydroxy-2-(4-hydroxy-phenyl)-benzo[b]thiophen-3-yl]-[4-(2- piperidin-1-yl-ethoxy)-phenyl]-methanone hydrate

### a) Preparation from 1-(2-{4-[6-hydroxy-2-(4-hydroxy-phenyl)-benzo[b]thiophene-3-carbonyl]-phenoxy}-ethyl)-piperidinium chloride · 0.37 methanol · 0.51 methylene chloride:

98.0 g of 1-(2-{4-[6-hydroxy-2-(4-hydroxy-phenyl)-benzo[b]thiophene-3-carbonyl]-phenoxy}-ethyl)-piperidinium chloride - 0.37 methanol · 0.51 methylene chloride (88.49 g, 173.5 mmol, calcd. dry) prepared according to example 7 item b were treated with aq. sodium hydroxide (2 N, 415 mL, 830 mmol) and acetone (580 mL) at ambient temperature with stirring. Acetic acid (aq., 2 N, 330 mL, 660 mmol) was then added slowly (approx. 0.75 h) at 16 - 18 °C to adjust a pH of 8. After seeding, the resulting suspension was stirred for 1 h at room temperature °C and filtered. The solid was washed with water (10 x 50 mL).
A sample (1.0 g) was dried at 60 °C under reduced pressure to yield 0.76 g of [6-Hydroxy-2-(4-hydroxy-phenyl)-benzo[b]thiophen-3-yl]-[4-(2-piperidin-1-yl-ethoxy)-phenyl]-methanone · 0.98 H₂O · 0.36 acetone. Yield: quantitative. **Purity**: 99.6 % (HPLC). **H₂O**: 3.46 % by weight, corresponding to approx. 0.98 molar equivalents (KFT). **Aceton**: 4.03 % by weight, corresponding to approx. 0.36 molar equivalents (GC). **Mp**.: 126.7 - 129.8 °C. **DSC**: Peak at 119.89 °C. **¹H NMR** (300 MHz, DMSO-d₆, ppm): δ 9.75 (b, 2 H, -OH), 7.65 (d, 2 H, arom.), 7.34 (d, 1 H, arom.), 7.25 (d, 1 H, arom.), 7.18 (d, 2 H, arom.), 6.91 (d, 2 H, arom.), 6.86 (dd, 1 H, arom.), 6.68 (d, 2 H, arom.), 4.06 (t, 2 H, -OCH₂CH₂NR₂), 2.60 (t, 2 H, -OCH₂CH₂NR₂), 2.37 (m, 4 H, piperidine), 1.60 - 1.22 (m, 6 H, piperidine). **¹³C-{H}-NMR** (75 MHz, DMSO-d₆, ppm): δ 192.50 (>C=O), 162.78 (=C<), 157.80 (=C<), 155.39 (=C<), 140.26 (=C<), 139.19 (=C<), 132.26 (=C<), 131.71 (=CH-), 129.73 (=C<), 129.65 (=C<), 129.62 (=CH-), 123.75 (=C<), 123.25 (=CH-), 115.64 (=CH-), 115.14 (=CH-), 114.45 (=CH-), 107.07 (=CH-), 65.96 (-CH₂-), 57.05 (-CH₂-), 54.27 (-CH₂-), 25.49 (-CH₂-), 23.83 (-CH₂-). **IR** (KBr, cm⁻¹): 3194, 2935, 1715, 1627, 1592, 1543, 1500, 1469, 1350, 1314, 1259, 1170, 1129, 1033, 1024, 943, 909, 836, 791, 764, 646, 637, 534. **LC/MS** (ESI(+), RT; 10.18 min): m/z = 474.0 [MH-H₂O]⁺. **XRPD**the X-ray diffractogram exhibits signals at the following 2θ values:

| **2theta[°]** | **l/l₀** |
|---|---|
| 8.13 | 543.33 |
| 9.54 | 105.35 |
| 12.46 | 177.74 |
| 13.13 | 195.6 |
| 13.93 | 190.27 |
| 15.18 | 279.6 |
| 16.15 | 136.11 |
| 16.9 | 116.75 |
| 17.9 | 95.21 |
| 18.11 | 206.09 |
| 18.39 | 416.55 |
| 18.65 | 444.52 |
| 19.44 | 117.96 |
| 20.63 | 122.65 |
| 21.84 | 188.62 |
| 22.08 | 111.68 |
| 22.43 | 149.95 |
| 23.07 | 1000 |
| 23.49 | 390.77 |
| 23.76 | 559.27 |
| 24.63 | 134.32 |
| 24.95 | 88.15 |
| 26.02 | 203.01 |
| 26.32 | 92.73 |
| 26.49 | 101.78 |
| 28.08 | 98.79 |
| 31.25 | 83.6 |
| 31.69 | 87.04 |
| 35.33 | 84.36 |
| 40.36 | 91.82 |

### b) Preparation from 1-(2-{4-[6-hydroxy-2-(4-hydroxy-phenyl)-benzo[b]thiophene-3-carbonyl]-phenoxy}-ethyl)-piperidinium bromide · 0.25 methanol · 0.52 methylene chloride:

1-(2-{4-[6-hydroxy-2-(4-hydroxy-phenyl)-benzo[b]thiophene-3-carbonyl]-phenoxy}-ethyl)-piperidinium bromide · 0.25 methanol · 0.52 methylene chloride (11.8 g, 21.3 mmol) prepared according to example 9 item a was treated with sodium hydroxide (aq., 2 N, 47 mL, 94 mmol) and acetone (70 mL) at ambient temperature with stirring. Acetic acid (aq., 2N, 42 mL, 84 mmol) was added slowly (approx. 1 h) at 12 °C to adjust a pH of 8 - 9. After seeding, the resulting suspension was stirred for 0.5 h at 8 -13 °C and filtered. The solid was washed with a mixture of water and acetone (2:1, 7 x 10 mL) and cold acetone (2x10 mL) and dried at 45 °C under reduced pressure (20 mbar) until constancy of weight to yield 10.6 g of [6-Hydroxy-2-(4-hydroxy-phenyl)-benzo[b]thiophen-3-yl]-[4-(2-piperidin-1-yl-ethoxy)-phenyl]-methanone · 1.10 H₂O · 0.67 acetone. Yield: quantitative. **Purity**: 97.16 %. **Water**: 3.69 % by weight, corresponding to approx. 1.10 molar equivalents (KFT). **Aceton**: 7.36 % by weight, corresponding to approx. 0.67 molar equivalents (GC). **Assay**: 96.3 % (HCl^{aq.}). **DSC**: Peak at 118.43 °C. **IR** (KBr, cm⁻¹): 3192, 2935, 1716, 1627, 1593, 1542, 1499, 1469, 1351, 1314, 1259, 1170, 1129, 1033, 943, 909, 836, 791, 764, 646, 637, 628, 534.

Subsequent dryings of [6-Hydroxy-2-(4-hydroxy-phenyl)-benzo[b]thiophen-3-yl]-[4-(2-piperidin-1-yl-ethoxy)-phenyl]-methanone **·** 1.10 H₂O · 0.67 acetone, carried out at different conditions respectively, resulted in the following changes concerning the content of H₂O:

| Subsequent drying conditions: | % by weight H₂O (KFT): |
|---|---|
| 70 °C at 20 mbar for 3 h | 3.25 |
| 110 °C at atmospheric pressure for 25 min. | 0.77 |

### Example 7

### Solvates of 1-(2-{4-[6-Hydroxy-2-(4-hydroxy-phenyl)-benzo[b]thiophene-3-carbonyl]-phenoxy}-ethyl)-piperidinium chloride

### a) 1-(2-{4-[6-Hydroxy-2-(4-hydroxy-phenyl)-benzo[b]thiophene-3-carbonyl]-phenoxy}-ethyl)-piperidinium chloride · 0.28 methanol · 0.53 methylene chloride:

A solution of boron trichloride in methylene chloride (1 M, 446 mL, 446 mmol) was added dropwise to a mixture of 1-(2-{4-[6-Methoxy-2-(4-methoxy-phenyl)-benzo[b]thiophene-3-carbonyl]-phenoxy}-ethyl)-piperidinium chloride (80.0 g, 0.149 mol), prepared as described under example 4 item e, and methylene chloride (400 mL) over a period of 3 h at 0 - 2 °C with stirring. The mixture was stirred for 1 h at 2 - 6 °C and heated to ambient temperature over a period of 2 h. Stirring was continued for approx. 18 h after which the suspension was cooled to 4 °C and boron trichloride in methylene chloride (1 M, 149 mL, 149 mmol) was added within 1.5 h, not exceeding an inner temperature of 7 °C. The suspension was heated at reflux for 6.6 h and cooled to room temperature. Boron trichloride in methylene chloride (1 M, 149 mL, 149 mmol) was added over a period of 1 h at 22 °C after which the mixture was heated at reflux for 5 h with stirring. The mixture was cooled to 15 °C and methanol (240 mL) was added slowly (0.75 h), not exceeding an inner temperature of 27 °C. The resulting suspension was heated at reflux for 0.5 h and allowed to reach room temperature. Stirring was continued for a further 60 min and the precipitate was filtered off. The crystalline solid was washed with methanol (6 x 75 mL). A sample (79.1 g) was dried at 50 °C under reduced pressure (40 mbar) to give 66.78 g of 1-(2-{4-[6-Hydroxy-2-(4-hydroxy-phenyl)-benzo[b]thiophene-3-carbonyl]-phenoxy}-ethyl)-piperidinium chloride 0.28 methane) 0.53 methylene chloride.
Yield: 92.6 % (138 mmol, calcd. dry). **Purity**: 98.18 % (HPLC). **Methanol**: 1.6 % by weight, corresponding to approx. 0.28 molar equivalents (GC). **Methylen chloride**: 8.0 % by weight, corresponding to approx. 0.53 molar equivalents (GC). **Mp**.: 225.8 °C; DSC: first peak at 201.4 °C, second peak at 225.3 °C, third peak at 262.9 °C, fourth peak at 338.5 °C; **¹H NMR** (300 MHz, DMSO-d₆, ppm): δ 10.68 (bs, 1 H, -NH), 9.88 (bs, 2 H, -OH), 7.70 (d, 2 H, arom.), 7.38 (m, 1 H, arom.), 7.27 (d, 1 H, arom.), 7.17 (d, 2 H, arom.), 6.97 (d, 2 H, arom.), 6.89 (m, 1 H, arom.), 6.71 (d, 2 H, arom.) 5.75 (CH₂Cl₂), 4.45 (m, 2H, -OCH₂CH₂NH-), 3.51 - 3.37 (m, 4 H, -OCH₂CH₂NH-, piperidine), 3.17 (CH₃OH), 2.95 (m, 2 H, piperidine,) 1.95 -1.22 (m, 6 H, piperidine). **¹³C-{H}-NMR** (75 MHz, DMSO-d₆, ppm): δ 192.54 (>C=O), 161.60 (=C<), 157.89 (=C<), 155.50 (=C<), 140.59 (=C<), 139.20 (=C<), 132.16 (=C<), 131.73 (=CH-), 130.35 (=C<), 129.64 (=CH-), 129.55 (=C<), 123.68 (=C<), 123.19 (=CH-), 115.70 (=CH-), 115.23 (=CH-), 114.61 (=CH-), 107.11 (=CH-), 62.51 (-CH₂-), 54.45 (-CH₂-), 52.55 (-CH₂-), 22.23 (-CH₂-), 21.08 (-CH₂-)- **IR** (KBr, cm⁻¹): 3203, 2958, 2722, 1653 (ν_{C=O}), 1597, 1548, 1501, 1467, 1437, 1419, 1343, 1309, 1250, 1166, 1065, 1037, 1021, 953, 939, 907, 840, 817, 808, 786, 738, 645, 635, 627, 594, 524. **LC/MS** (ESI(+), RT; 10.31 min): m/z = 473.9 [M-Cl⁻]⁺. **XRPD**: the X-ray diffractogram exhibits signals at the following 2θ values:

| **2theta[°]** | **l/l₀** |
|---|---|
| 11.01 | 290.35 |
| 13.31 | 167.25 |
| 13.46 | 631.03 |
| 15.88 | 208.55 |
| 16.45 | 1000 |
| 17.56 | 861.31 |
| 18.76 | 994.61 |
| 18.9 | 439.77 |
| 19.1 | 781.29 |
| 19.53 | 949.56 |
| 19.76 | 612.2 |
| 20.75 | 505.2 |
| 20.98 | 478.97 |
| 21.26 | 615.03 |
| 21.61 | 180 |
| 23.4 | 498.84 |
| 23.57 | 774.29 |
| 24.4 | 465.6 |
| 24.76 | 328.79 |
| 25.32 | 396.14 |
| 25.42 | 182.71 |
| 25.84 | 375.3 |
| 25.96 | 449.3 |
| 28.69 | 208.41 |
| 28.73 | 204.75 |
| 28.78 | 210.11 |
| 29.39 | 287.22 |
| 31.1 | 176.01 |
| 31.17 | 182.21 |
| 32.74 | 166.22 |

### b) 1-(2-{4-[6-Hydroxy-2-(4-hydroxy-phenyl)-benzo[b]thiophene-3-carbonyl]-phenoxy}-ethyl)-piperidinium chloride · 0.37 methanol · 0.51 methylene chloride:

Boron trichloride in methylene chloride (1 M, 149 mL, 149 mmol) was cooled to 2 - 5 °C and 1-(2-{4-[6-Methoxy-2-(4-methoxy-phenyl)-benzo[b]thio-phene-3-carbonyl]-phenoxy}-ethyl)-piperidinium chloride (20.0 g, 37.2 mmol) was added in portions over a period of 70 min with stirring. The mixture was stirred for 35 min at 3 - 4 °C and allowed to warm to ambient temperature within 90 min. Stirring was continued for 49 h after which the mixture was cooled to 12 °C. Methanol (60 mL) was then added slowly (approx. 20 min) and the resulting suspension was heated at reflux for 0.75 h. The solution was allowed to reach 20 °C and the solid was filtered off. The crystalline residue was with methanol (6x10 mL) and dried at 45°C under reduced pressure to give 1-(2-{4-[6-Methoxy-2-(4-methoxy-phenyl)-benzo[b]thiophene-3-carbonyl]-phenoxy}-ethyl)-piperidinium chloride · 0.37 methanol · 0.51 methylene chloride. Yield: 20.14 g (34.9 mmol, 95.0 %, calcd.). **Purity**: HPLC: 98.01 % (HPLC). **Methanol**: 2.1 % by weight, corresponding to approx. 0.37 molar equivalents (GC). **Methylene chloride**: 7.7 % by weight, corresponding to approx. 0.51 molar equivalents (GC). **Assay**: 101.7 % (AgNO₃).
**Mp**.: 213 - 221 °C. **DSC**: Peak at 197.47 °C, Peak at 221.81 °C, Peak at 334.13 °C. **IR** (KBr, cm⁻¹): 3205, 2958, 2722, 2558, 1653 (ν_{C=O}), 1597, 1547, 1500, 1467, 1437, 1418, 1343, 1309, 1250, 1166, 1065, 1037, 1020, 953, 938, 907, 840, 816, 808, 785, 738, 645, 635, 627, 594, 524. **XRPD**: XRD data correspond to those given for Example 7 item a).

### c) 1-(2-{4-[6-Hydroxy-2-(4-hydroxy-phenyl)-benzo[b]thiophene-3-carbonyl]-phenoxy}-ethyl)-piperidinium chloride · 0.34 methanol · 0.62 methylene chloride from non-solvated crystalline 1-(2-{4-[6-Hydroxy-2-(4-hydroxy-phenyl)-benzo[b]thiophene-3-carbonyl]-phenoxy}-ethyl)-piperidinium chloride:

A mixture of 1-(2-{4-[6-Hydroxy-2-(4-hydroxy-phenyl)-benzo[b]-thiophene-3-carbonyl]-phenoxy}-ethyl)-piperidinium chloride (12.1 g, 0.024 mol), prepared as described under example 8 item a, methanol (350 mL) and methylene chloride (230 mL) were heated to reflux. The resulting clear solution was allowed to cool to 45 °C and concentrated under reduced pressure. The resulting suspension was cooled to 2 °C with stirring. The solid was isolated, washed with a mixture of methanol and methylene chloride (60:40 v:v, 3 x 4 mL) and dried at 45 °C under reduced pressure (40 mbar) to give 1-(2-{4-[6-Hydroxy-2-(4-hydroxy-phenyl)-benzo[b]thiophene-3-carbonyl]-phenoxy}-ethyl)-piperidinium chloride · 0.34 methanol . 0.62 methylene chloride. Yield: 66 %. **Purity**: 99.10 % (HPLC). **Methanol**: 1.9 % by weight, corresponding to approx. 0.34 molar equivalents (GC). **Methylene chloride**: 9.2 % by weight, corresponding to approx. 0.62 molar equivalents (GC). **Mp**..: 255 - 259.1 °C **DSC**: first peak at 206.2 °C, second peak at 223.3 °C, third peak at 233.1 °C, fourth peak at 268.8 °C, fifth peak at 343.3 °C. IR (KBr, cm⁻¹): 3202, 2958, 2722, 1653 (ν_{C=O}), 1596, 1549, 1500, 1467, 1437, 1418, 1343, 1309, 1270, 1227, 1166, 1065, 1036, 1020, 953, 939, 907, 839, 816, 808, 785, 738, 645, 635, 627, 594, 524. XRPD . XRD data correspond to those given for Example 7 item a).

### d) 1-(2-{4-[6-Hydroxy-2-(4-hydroxy-phenyl)-benzo[b]thiophene-3-carbonyl]-phenoxy}-ethyl)-piperidinium chloride · 0.08 methanol · 0.29 toluene:

1-(2-{4-[6-Hydroxy-2-(4-hydroxy-phenyl)-benzo[b]thiophene-3-carbonyl]-phenoxy}-ethyl)-piperidinium chloride (60.0 g, 0.118 mol), prepared as described under example 8 item a was dissolved in a mixture of water (540 mL) and aq. sodium hydroxide (cc., 20 mL, 0.38 mol) and washed with toluene (4 x 500 mL). The aqueous phase was treated with methanol (550 mL) and hydrochloric acid (aq., cc., 37 mL, 444 mmol) was added dropwise at approx. 15 °C to adjust a pH of 2 with stirring. The resulting suspension was heated to reflux and treated with a mixture of methanol and water (50:50 v/v, 300 mL) and cooled to 2 °C with stirring. The solid was collected by filtration, washed with cold aqueous methanol (50:50 v/v, 2 x 25 mL), water (2 x 25 mL), and cold aqueous methanol (50:50 v/v, 2 x 25 mL). A sample (3.21 g) was dried at 50 °C under reduced pressure (40 mbar) to give 1-(2-{4-[6-hydroxy-2-(4-hydroxy-phenyl)-benzo[b]thiophene-3-carbonyl]-phenoxy}-ethyl)-piperidinium chloride · 0.08 methanol 0.29 toluene. Yield: 51.2 g (100.4 mmol, 94.5 %, calc. dry). **Purity**: 98.94 % (HPLC). **Methanol**: 0.5 % by weight, corresponding to approx. 0.08 molar equivalents (GC). **Toluene**: 4.9 % by weight, corresponding to approx. 0.29 molar equivalents (GC). **Mp**.: 260.6 °C; **DSC**: first peak at 211.7 °C, second peak at 247.4, third peak at 267.0 °C, fourth peak at 332.9 °C. **IR** (KBr, cm⁻¹): 3203, 2958, 2722, 1653 (ν_{C=O}), 1597, 1548, 1501, 1467, 1437, 1419, 1343, 1309, 1250, 1166, 1065, 1037, 1021, 953, 939, 907, 840, 817, 808, 786, 738, 645, 635, 627, 594, 524. **XRPD**: the X-ray diffractogram exhibits signals at the following 2θ values:

| **2theta[°]** | **l/l₀** |
|---|---|
| 11.12 | 294.09 |
| 13.45 | 740.54 |
| 14.43 | 669.08 |
| 15.75 | 413.53 |
| 16.38 | 773.84 |
| 17.52 | 491.55 |
| 18.34 | 861.17 |
| 18.61 | 484.9 |
| 19.11 | 739.17 |
| 19.4 | 372.71 |
| 19.74 | 745.12 |
| 20.38 | 599.01 |
| 20.64 | 404.98 |
| 20.95 | 558.87 |
| 21.18 | 769.79 |
| 21.37 | 391.36 |
| 22.66 | 1000 |
| 23 | 268.21 |
| 23.47 | 419.87 |
| 23.72 | 667.65 |
| 24.1 | 315.09 |
| 24.17 | 356.16 |
| 24.36 | 305.51 |
| 25.08 | 513.78 |
| 25.51 | 217.57 |
| 25.64 | 477.11 |
| 25.89 | 257.51 |
| 28 | 268.99 |
| 29.02 | 239.51 |
| 29.28 | 222.56 |

### Example 8

### Non-solvated crystalline 1-(2-{4-[6-Hydroxy-2-(4-hydroxy-phenyl)-benzo[b]thiophene-3-carbonyl]-phenoxy}-ethyl)-piperidinium chloride

### a) Preparation by using a mixture of methanol/water (70:30 v/v) as the solvent:

1-(2-{4-[6-hydroxy-2-(4-hydroxy-phenyl)-benzo[b]thiophene-3-carbonyl]-phenoxy}-ethyl)-piperidinium chloride · 0.34 methanol · 0.62 toluene, (44.14 g, 0.0865 mol, calcd. dry), was dissolved in hot aqueous methanol (70:30 v/v, 610 mL). Approx. 50 mL of methanol was distilled off at 70 °C and a mixture of activated charcoal (3 g) and aqueous methanol (70:30 v/v, 50 mL) was added. The suspension was diluted with methanol (50 mL) and heated reflux to reflux. Insolubles were filtered off and the residue was washed with hot aqueous methanol (70:30 v/v, 10 mL). The combined filtrates were cooled 2 °C and stirring was continued for 2.3 h at 2 - 8 °C. The resulting solid was isolated and washed with aqueous methanol (70:30 v/v, 3 x 20 mL) to give 48.23 g of 1-(2-{4-[6-hydroxy-2-(4-hydroxy-phenyl)-benzo[b]thiophene-3-carbonyl]-phenoxy}-ethyl)-piperidinium chloride. Yield: 31.22 g (70.8 %, calcd. dry). **Purity**: 99.59 % (HPLC).

A sample (46.5 g) of the product as obtained above (30.1 g, 0.059 mol, calcd. dry) was dissolved in hot aqueous methanol (70:30 v/v, 330 mL) and a mixture of activated charcoal (3.0 g) in aqueous methanol (70:30 v/v, 30 mL) was added. The mixture was heated to reflux and insolubles were filtered off. The residue was washed with aqueous methanol (70:30 v/v, 10 mL). The combined filtrates were cooled to 2 °C and stirring was continued for a further 60 min at 2 - 5 °C. The solid was isolated, washed with cold aqueous methanol (70:30 v/v, 3 x 15 mL) and dried under reduced pressure for (50 °C, 7 h). Yield: 22.93 g (44.96 mmol, 76.1 %). **Purity**: 99.70 %; all individual impurities: nmt 0.10 % (HPLC). **Water**: 0.16 % (KFT). **Assay** (NaOH): 99.9 %. **Assay (AgNO₃)**: 99.8 %. **Sulphated ash**: 0.02 %. **Heavy metals**: nmt 20 ppm. **Residual solvents**: methanol (707 ppm). **Mp**.: 260.0 °C. **DSC**: first peak at 266.1 °C, second peak at 331.7 °C. **¹H NMR** (300 MHz, DMSO-d₆, ppm): δ 10.68 (b, 1 H, -NH⁺), 9.87 (m, 2 H, -OH), 7.70 (d, ³J = 9.0 Hz, 2 H, arom.), 7.38 (d, ⁴J = 2.2 Hz, 1 H, arom:), 7.26 (d, ³J = 8.8 Hz, 1 H, arom.), 7.17 (d, ³J = 8.7 Hz, 2 H, arom.), 6.96 (d, ³J = 9.0 Hz, 2 H, arom.), 6.88 (dd, ³J = 8.8 Hz, ⁴J = 2.3 Hz, 1 H, arom.), 6.71 (d, ³J = 8.7 Hz, 2 H, arom.), 4.45 (m, 2 H, -OCH₂CH₂NH-), 3.42 (m, 4 H, -NHCH₂CH₂CH₂-, -OCH₂CH₂NH-), 2.97 (m, 2 H, -NHCH₂CH₂CH₂-), 1.87 - 1.28 (m, 6 H, -CH₂CH₂CH₂-NR₂, -CH₂CH₂CH₂-);
**¹³C-{H}-NMR** (75 MHz, DMSO-d₆, ppm): δ 192.57 (>C=O), 161.64 (=C<), 157.93 (=C<), 155.53 (=C<), 140.61 (=C<), 139.22 (=C<), 132.19 (=C<), 131.77 (=CH-), 130.36 (=C<), 129.67 (=CH-), 129.57 (=C<), 123.70 (=C<), 123.22 (=CH-), 115.74 (=CH-), 115.27 (=CH-), 114.65 (=CH-), 107.14 (=CH-), 62.54 (-CH₂-), 54.47 (-CH₂-), 52.57 (-CH₂-), 22.26 (-CH₂-), 21.12 (-CH₂-). **IR** (KBr, cm⁻¹): 3146, 2945, 2694, 1643 (ν_{C=O}), 1597 (ν_{arom.}), 1540, 1500, 1468 (ν_{arom.}), 1358, 1259, 1170, 1038, 1002, 906, 840, 807, 623. **XRPD**: the X-ray diffractogram exhibits signals at the following 2θ values:

| **2theta[°]** | **l/l₀** |
|---|---|
| 12.78 | 150.93 |
| 14.45 | 696.72 |
| 15.75 | 372.19 |
| 16.26 | 158 |
| 18.61 | 140.55 |
| 19.13 | 332.59 |
| 19.62 | 95.86 |
| 19.78 | 226.51 |
| 20.41 | 155.52 |
| 21 | 344.2 |
| 21.18 | 715.36 |
| 21.38 | 488.6 |
| 21.65 | 139.21 |
| 21.87 | 131.02 |
| 22.48 | 206.29 |
| 22.67 | 1000 |
| 22.88 | 136.09 |
| 23.02 | 331.41 |
| 23.82 | 100.83 |
| 24.06 | 222.51 |
| 24.4 | 171.37 |
| 25.09 | 114:33 |
| 25.47 | 137.89 |
| 25.79 | 208.69 |
| 25.9 | 174.26 |
| 27.63 | 142.62 |
| 27.98 | 107.44 |
| 29.02 | 222.22 |
| 31.28 | 158.82 |
| 31.9 | 99.36 |

### b) Preparation by using a mixture of 1-propanol/water (75:25 v/v) as the solvent:

1-(2-{4-[6-Hydroxy-2₋(4-hydroxy-phenyl)-benzo[b]thiophene-3-carbonyl]-phenoxy}-ethyl)-piperidinium chloride was also obtained in a similar manner as described under example 8 item a, employing 1-(2-{4-[6-Hydroxy-2-(4-hydroxy-phenyl)-benzo[b]thiophene-3-carbonyl]-phenoxy}-ethyl)-piperidinium chloride · 0.28 methanol · 0.53 methylene chloride, obtained according to example 7 item a, and a mixture of 1-propanol and water (75:25 v/v) as solvent. Yield: 34 %. **Purity**: 99.28 % (HPLC). **DSC**: first peak at 266.3 °C, second peak at 335.4°C). **IR** (KBr, cm⁻¹): 3146, 2945, 2692, 1643 (ν_{C=O}), 1597, 1541, 1500, 1468, 1431, 1357, 1311, 1259, 1169, 1125, 1078, 1048, 1038, 1002, 952, 906, 840, 807, 762, 724, 706, 643, 623, 588, 529.

### Example 9

### Solvates of 1-(2-(4-[6-Hydroxy-2-(4-hydroxy-phenyl)-benzo[b]thiophene-3-carbonyl]-phenoxy)-ethyl)-piperidinium bromide

### a) 1-(2-{4-[6-Hydroxy-2-(4-hydroxy-phenyl)-benzo[b]thiophene-3-carbonyl]-phenoxy}-ethyl)-piperidinium bromide · 0.25 methanol · 0.52 methylene chloride:

Boron tribromide (51.6 g, 0.206 mol) in methylene chloride (20 mL) was added to a mixture of 1-(2-{4-[6-Methoxy-2-(4-methoxy-phenyl)-benzo[b]thiophene-3-carbonyl]-phenoxy}-ethyl)-piperidinium bromide (40.0 g, 0.0687 mol), prepared as described under example 5 item a, and methylene chloride (560 mL) over a period of 2 h at 37 - 39 °C with stirring. Stirring was continued for 0.5 h at 39 °C and the mixture was cooled to 17 °C. Methanol (111 mL) was added slowly (0.5 h) at 17 - 32 °C and the resulting suspension was heated at reflux for 0.25 h. The suspension was allowed to cool to 15 - 20 °C and stirring was continued for 15 min. The solid was filtered off, washed with methanol (4 x 35 mL) and dried under reduced pressure (45 °C, 30 mbar, 3 h) to give 1-(2-{4-[6-Hydroxy-2-(4-hydroxy-phenyl)-benzo[b]thiophene-3-carbonyl]-phen-oxy}-ethyl)-piperidinium bromide · 0.25 methanol · 0.52 methylene chloride. Yield: 33.49 g (55.17 mmol, 80.4 %). **Purity**: 97.29 % (HPLC). **Methanol**: 1.3 % by weight, corresponding to approx. 0.25 molar equivalents (GC). **Methylene chloride**: 7.3 % by weight, corresponding to approx. 0.52 molar equivalents (GC). **Mp.**: 250.4 °C. **Water**: 0.36 % (KFT). Assay (AgNO₃): 99.4 % (calcd. on non-solvated substance) **DSC**: first peak at 182.6 °C, second peak at 197.9 °C, third peak at 254.4 °C, fourth peak at 352.2 °C. **IR** (KBr, cm⁻¹): 3247, 2945, 2742, 1653 (ν_{C=O}), 1597, 1546, 1500, 1467, 1433, 1418, 1341, 1305, 1250, 1226, 1167, 1064, 1037, 1019, 952, 907, 839, 817, 808, 784, 738, 627, 523. **XRPD**: the X-ray diffractogram exhibits signals at the following 20 values:

| **2theta[°]** | **l/l₀** |
|---|---|
| 10.82 | 183.94 |
| 13.47 | 450.42 |
| 14.11 | 305.45 |
| 15.92 | 415.8 |
| 16.43 | 524.47 |
| 17.11 | 198.42 |
| 17.57 | 955.66 |
| 18.76 | 750.64 |
| 18.96 | 592.19 |
| 19.06 | 805.32 |
| 19.52 | 300.68 |
| 19.75 | 280.67 |
| 20 | 186.3 |
| 20.73 | 251.74 |
| 20.98 | 665.41 |
| 21.18 | 984.83 |
| 23.06 | 224.76 |
| 23.39 | 503.31 |
| 23.54 | 999.33 |
| 24.37 | 476.01 |
| 24.53 | 219.79 |
| 25.28 | 541.05 |
| 25.88 | 411.61 |
| 28.55 | 158.07 |
| 28.64 | 179.27 |
| 28.74 | 186.54 |
| 29.4 | 249.82 |
| 31.07 | 257.25 |
| 31.71 | 218.43 |
| 32.68 | 153.25 |

### b) 1-(2-{4-[6-Hydroxy-2-(4-hydroxy-phenyl)-benzo[b]thiophene-3-carbonyl]-phenoxy}-ethyl)-piperidinium bromide · 0.08 methanol · 0.51 methylene chloride:

A flask was charged with non-solvated 1-(2-{4-[6-Hydroxy-2-(4-hydroxy-phenyl)-benzo[b]thiophene-3-carbonyl]-phenoxy}-ethyl)-piperidinium bromide (4.5 g, 0.008 mol) and a mixture of methanol and methylene chloride (1:1 v/v, 115 mL). The mixture was heated to reflux. The resulting clear solution was allowed to cool to 45 °C. The suspension was concentrated under reduced pressure (870 mbar) at 45 °C and cooled to 2 3 °C with stirring. The solid was isolated, washed a mixture of methanol and methylene chloride (1:1 v/v, 3 x 3 mL), and dried at 50 °C under reduced pressure to to give 1-(2-{4-[6-Hydroxy-2-(4-hydroxy-phenyl)-benzo[b]thiophene-3-carbonyl]-phen-oxy}-ethyl)-piperidinium bromide · 0.08 methanol · 0.51 methylene chloride. Yield: 3.39 g (5.6 mmol, 70 %). **Purity**: 99.36 % (HPLC).
**Water**: 0.41 % (KFT). **Methanol**: 0.4 % by weight, corresponding to approx. 0.08 molar equivalents (GC). **Methylene chloride**: 7.3 % by weight, corresponding to approx. 0.51 molar equivalents (GC). **Mp**.: 256.5 - 258.4 °C; **DSC**: first peak at 180.1 °C, second peak at 199.6· °C, third peak at 219.7 °C, fourth peak at 263.9 °C, fifth peak at 359.2. **IR** (KBr, cm⁻¹): 3246, 2946, 2744, 2549, 1653 (ν_{C=O}), 1597, 1547, 1500, 1467, 1434, 1418, 1339, 1305, 1249, 1226, 1167, 1064, 1037, 1019, 952, 937, 907, 852, 839, 818, 808, 785, 739, 660, 645, 635, 627, 594, 524. **XRPD**: XRD data correspond to those given for Example 9 item a).

### c) 1-(2-{4-[6-Hydroxy-2-(4-hydroxy-phenyl)-benzo[b]thiophene-3-carbonyl]-phenoxy}-ethyl)-piperidinium bromide · 0.51 toluene:

Non-solvated 1-(2-{4-[6-Hydroxy-2-(4-hydroxy-phenyl)-benzo[b]thiophene-3-carbonyl]-phenoxy}-ethyl)-piperidinium bromide (2.2 g, 3.97 mmol), prepared according to example 10. item b, was dissolved in a mixture of methanol and water (70:30 v/v, 18.4 mL) at 70 °C. Toluene (6 mL) was added and the mixture was concentrated under reduce pressure. This procedure was repeated once using 2 mL of toluene upon which the product crystallized. Toluene (10 mL) was then added to the suspension and the mixture was evaporated to dryness. The solid was dried at 55 °C under reduced pressure (40 mbar) to give 2.4 g (3.99 mmol) of the title compound. Yield: quantitative. Purity: 97.92 % (HPLC). **Toluene**: 7.75 % by weight, corresponding to approx. 0.51 molar equivalents (GC). **Water**: 0.14 % (KFT). **Mp**.: 256.4 - 258.1 °C. **DSC**: peak at 263.11 °C, peak at 347.85° **IR** (KBr, cm⁻¹): 3243, 2941, 2738, 1653 (ν_{C=O}), 1595, 1550, 1501, 1467, 1432, 1416, 1340, 1309, 1249, 1224, 1165, 1064, 1035, 1018, 952, 936, 907, 839, 822, 808, 785, 728, 695, 644, 635, 626, 523. **XRPD**: the X-ray diffractogram exhibits signals at the following 2θ values:

| **2theta[°]** | **l/l₀** |
|---|---|
| 13.34 | 549.23 |
| 13.98 | 295.54 |
| 15.74 | 469.13 |
| 16.29 | 622.91 |
| 17.11 | 270.99 |
| 17.4 | 345.69 |
| 17.55 | 682.27 |
| 18.2 | 928.25 |
| 18.46 | 543.02 |
| 18.85 | 356.16 |
| 18.97 | 478.95 |
| 19.23 | 320.72 |
| 19.61 | 360.95 |
| 20.22 | 323.43 |
| 20.5 | 806.91 |
| 20.79 | 548.86 |
| 21.1 | 483.12 |
| 22.59 | 326.37 |
| 23.32 | 548.49 |
| 23.53 | 1000 |
| 23.96 | 527.24 |
| 24.18 | 283.44 |
| 24.88 | 489.08 |
| 25.04 | 256.1 |
| 25.4 | 477.86 |
| 25.5 | 551.08 |
| 29.38 | 324.98 |
| 30.33 | 325.03 |
| 31.42 | 300.35 |
| 33.63 | 258.85 |

### d) 1-(2-{4-[6-Hydroxy-2-(4-hydroxy-phenyl)-benzo[b]thiophene-3-carbonyl]-phenoxy}-ethyl)-piperidinium bromide · 0.16 methanol · 0.64 chlorobenzene

Boron tribromide (21.5 mL, 85.8 mmol) in chlorobenzene (20 mL) was added slowly (40 min) to a mixture of 1-(2-{4-[6-Methoxy-2-(4-methoxy-phenyl)-benzo[b]thiophene-3-carbonyl]-phenoxy}-ethyl)-piperidinium bromide (10.0 g, 17.2 mmol) and chlorobenzene (80 mL) at 2 - 3 °C with stirring. The mixture was allowed to reach room temperature and stirring was continued for 44.5 h. Methanol (30 mL) was added slowly at 10 °C and the resulting suspension was allowed to reach room temperature. Stirring was continued for a further 60 min and the suspension was filtered. The solid was washed with methanol (6 x 5 mL) and dried at 45 °C under reduced pressure to give the title compound. Yield: 7:63 g (13.75 mmol, 80.5 %, calcd. dry). **Purity**: 95.71 %. **Methanol**: 0.83 % by weight, corresponding to approx. 0.16 molar equivalents (GC). **Chlorobenzene**: 11.41 % by weight, corresponding to approx. 0.64 molar equivalents (GC).

### e) 1-(2-{4-[6-Hydroxy-2-(4-hydroxy-phenyl)-benzo[b]thiophene-3-carbonyl]-phenoxy}-ethyl)-piperidinium chloride · 0.35 methanol · 0.67 methylene chloride

1-(2-{4-[6-Hydroxy-2-(4-hydroxy-phenyl)-benzo[b]thiophene-3-carbonyl]-phenoxy}-ethyl)-piperidinium chloride · 0.35 methanol 0.67 methylene chloride was obtained in a similar manner as described under example 9 item a), employing 1-(2-{4-[6-Methoxy-2-(4-methoxyphenyl)-benzo[b]thiophene-3-carbonyl]-phenoxy}-ethyl)-piperidinium bromide instead of 1-(2-{4-[6-Methoxy-2-(4-methoxy-phenyl)-benzo[b]thiophene-3-carbonyl]-phenoxy}-ethyl)-piperidinium chloride.
**Methanol**: 1.93 % by weight, corresponding to approx. 0.35 molar equivalents (GC).
**Methylene chloride**: 9.8 % by weight, corresponding to approx. 0.67 molar equivalents (GC).

### Example 10

### Non-solvated crystalline 1-(2-{4-[6-Hydroxy-2-(4-hydroxy-phenyl)-benzo[b]thiophene-3-carbonyl]-phenoxy}-ethyl)-piperidinium bromide

### a) Synthesis via [6-Hydroxy-2-(4-hydroxy-phenyl)-benzo[b]thiophen-3-yl]-[4-(2-piperidin-1-yl-ethoxy)-phenyl]-methanone hydrate

Moist [6-Hydroxy-2-(4-hydroxy-phenyl)-benzo[b]thiophen-3-yl]-[4-(2- piperidin-1-yl-ethoxy)-phenyl]-methanone hydrate (84.8 g, 172 mmol, calcd. dry) prepared according to example 6 item a, was suspended in a mixture of methanol (600 mL) and water (175mL) at 30 °C. Hydrobromic acid (aq., 48 %, 27.0 mL, 240.2 mmol) was added dropwise maintaining the temperature below 35 °C to adjust a pH of 1.5. The solution was heated to reflux and concentrated under normal pressure. The solution was allowed to reach room temperature and seeded with appropriate material. The resulting suspension was cooled to 1 °C and stirring was continued for approx. 30 min. The mixture was filtered and the solid was washed with a mixture of methanol and water (70:30 v/v, 3 x 25 mL) and dried at 60 °C under reduced pressure. Yield: 83.62 g of 1-(2-{4-[6-Hydroxy-2-(4-hydroxy-phenyl)-benzo[b]thiophene-3-carbonyl]-phenoxy}-ethyl)-piperidinium bromide (150.8 mmol,
87.4 %). Purity: 99.75 %; all individual impurities: nmt 0.10 % (HPLC). **Water**: 0.12 % (KFT). **Acetone**: 23 ppm (GC). **Loss on drying**: 0.04 %. **Heavy metals**: < 10 ppm. **Sulphated ash**: 0.04 %. **Assay**: 100.5 % (HClO₄), 99.9 % (AgNO₃). **¹H NMR** (300 MHz, DMSO-d₆, ppm): δ 9.72 (m, 2 H, -OH), 9.52 (b, 1 H, -NH⁺), 7.69 (d, 2 H, arom.), 7.36 (d, 1 H, arom.), 7.26 (d, 1 H, arom.), 7.17 (d, 2 H, arom.), 6.97 (d, 2 H, arom.), 6.86 (dd, 1 H, arom.), 6.69 (d, 2 H, arom.), 4.40 (m, 2 H, -OCH₂CH₂NH-), 3.48 (m, 4 H, -NHCH₂CH₂CH₂-, -OCH₂CH₂NH-), 3.00 (m, 2 H, -NHCH₂CH₂CH₂-), 1.87 - 1.22 (m, 6 H, -CH₂CH₂CH₂-NR₂, -CH₂CH₂CH₂-).
**¹³C-{H}-NMR** (75 MHz, DMSO-d₆, ppm): δ 192.53 (>C=O), 161.61 (=C<), 157.77 (=C<), 155.39 (=C<), 140.48 (=C<), 139.2 (=C<), 132.17 (=C<), 131.71 (=CH-), 130.33 (=C<), 129.64 (=CH-), 129.54 (=C<), 123.72 (=C<), 123.17 (=CH-), 115.66 (=CH-), 115.18 (=CH-), 114.64 (=CH-), 107.09 (=CH-), 62.37 (-CH₂-), 54.6 (-CH₂-), 52.67 (-CH₂-), 22.27 (-CH₂-), 20.97 (-CH₂-). **IR** (KBr, cm⁻¹): 3174, 2954, 1646 (ν_{C=O}), 1594, 1544, 1498, 1478, 1452, 1409, 1384, 1348, 1305, 1254, 1222, 1198, 1165, 1057, 1039, 1011, 995, 952, 926, 908, 857, 840, 831, 807, 674, 645, 632, 622, 588, 517. **LC/MS** (ESI(+), RT; 8.09 min): m/z = 473.8 [M-Br⁻]⁺.
**XRPD**: the X-ray diffractogram exhibits signals at the following 2θ values:

| **2theta[°]** | **l/l₀** |
|---|---|
| 12.32 | 179.64 |
| 14 | 450.33 |
| 14.68 | 124.93 |
| 15.02 | 334.5 |
| 18.31 | 139.86 |
| 19.14 | 467.48 |
| 19.28 | 319.2 |
| 19.7 | 259.65 |
| 20.93 | 828.37 |
| 21.22 | 262.49 |
| 21.91 | 125.76 |
| 22.47 | 339.99 |
| 22.6 | 1000 |
| 23.59 | 192 |
| 23.68 | 170.4 |
| 23.96 | 363.43 |
| 24.45 | 481.64 |
| 24.79 | 426.65 |
| 25.15 | 123.16 |
| 26.1 | 225.47 |
| 27.45 | 352.33 |
| 27.73 | 406.18 |
| 28.22 | 150.52 |
| 28.59 | 208.75 |
| 28.79 | 122.45 |
| 29.07 | 223.4 |
| 30.23 | 139.53 |
| 30.73 | 150.13 |
| 33.17 | 169.3 |
| 36.83 | 123.26 |

For an optional purification 1-(2-{4-[6-Hydroxy-2-(4-hydroxy-phenyl)-benzo[b]thiophene-3-carbonyl]-phenoxy}-ethyl)-piperidinium bromide (15.18 g, 27.4 mmol) as obtained above was dissolved in a mixture of water (40 mL) and methanol (95 mL). Activated charcoal (0.76 g) was added and the mixture was heated to reflux. Solids were filtered off and the residue was washed with a hot mixture of methanol (3.5 mL) and osmosis water (1.5 mL). The combined filtrates were cooled, seeded with appropriate material, and stirred for 1 h at 2 - 4 °C. The solid was isolated, washed (3 x) with a cold mixture of methanol (3.5 mL) and water (1.5 mL), and dried at 50 °C under reduced pressure. Yield: 12.45 g (22.5 mmol, 82.0 %):

### b) Synthesis via 1-(2-{4-[6-Hydroxy-2-(4-hydroxy-phenyl)-benzo[b]thiophene-3-carbonyl]-phenoxy}-ethyl)-piperidinium bromide · 0.16 methanol · 0.64 chlorobenzene

A sample (6.49 g, 11.7 mmol, calcd. dry) of the material obtained according to example 9 item d was dissolved in methanol (130 mL) and treated with activated charcoal (0.39 g). The mixture was heated to reflux, filtered, and the residue was washed with hot methanol (3 mL). The combined filtrates were cooled, seeded with appropriate material, and stored at 2 °C for 16.5 h. The solid was isolated, washed with 2 mL cold methanol (3 x 2 mL) and dried at 50°C under reduced pressure. Yield: 3.94 g (7.1 mmol, 60.7 %). **Purity**: 99.25 % (HPLC).

A sample of the material (3.13 g, 5.64 mmol) was re-crystallized from methanol (69 ml) and stirred for 1.75 h at -6 to -10 °C. The solid was isolated, washed with cold methanol (3 x 1 mL) and dried at 50°C under reduced pressure. Yield: 2.13 g (3.84 mmol, 68.3 %). **Purity**: 99.69 % (HPLC).

## Claims

1. A process for preparing a crystalline hydrate of a compound of formula XI 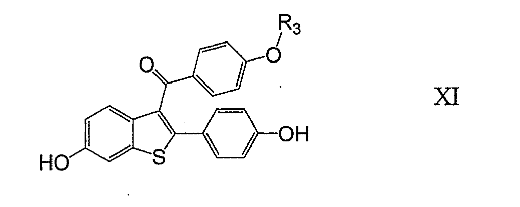 wherein R₃ is 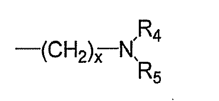 , wherein x = 1 to 20,
and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring,
wherein said process comprises the steps of:
a) providing a free base of the compound of formula XI, a salt of the compound of formula XI or a solvate form of a salt of the compound of formula XI comprising at least one solvated organic solvent,
b) dissolving said compound provided in step a) with a mixture of an antisolvent for a free base of a compound of formula XI and an aqueous solution of a proton acceptor in an at least stoichiometric molar amount or higher relative to the compound of formula XI provided in step a),
c) adjusting the pH of the mixture resulting from step b) to 7-9 by adding a proton donator,
d) optionally seeding the mixture resulting from step c) in order to induce crystallisation,
e) allowing hydrate of compound of formula XI to precipitate or to crystallize, optionally under agitation, and
f) separating the crystalline hydrate of compound of formula XI obtained in step e).

2. The process according to claim 1, wherein in R₃, x = 1 to 15, preferably 2 to 10, and in particular 2, and
R₄ and R₅ cooperatively with the N-atom form a part of a 5- to 7-membered ring, more preferably a 5- or 6-membered ring, in particular a 6-membered ring.

3. The process according to claim 1 or 2, wherein the hydrate of a compound of formula XI comprises 2.8 to 4.7 % by weight of water relative to a hydrate of a compound of formula XI, preferably 3.2 to 4.2 % by weight, more preferably 3.4 to 3.9 % by weight.

4. The process according to any one of claims 1 to 3, **characterized by** either one or a combination of the following features (g) to (k):
(g) said antisolvent for a free base of a compound of formula XI is acetone; preferably, the hydrate of compound of formula XI comprises said antisolvent in an amount of up to 15 % by weight relative to a hydrate of a compound of formula XI, preferably up to 9.2 % by weight, more preferably up to 7.4 % by weight and in particular 0.1 to 4.9 % by weight;
(h) the proton acceptor is selected from the group consisting of inorganic bases or organic amine bases, preferably alkaline metal hydroxides, earth alkaline metal hydroxides, ammonia and triethylamine, more preferably alkaline metal hydroxides, even more preferably sodium hydroxide and potassium hydroxide, and in particular sodium hydroxide;
(i) the at least stoichiometric amount of proton acceptor applied in step b) is 1 to 10 molar equivalents relative to-the compound of formula XI provided in step a), preferably 1.5 to 8 molar equivalents, more preferably 3 to 7 molar equivalents and in particular 4.2 to 6 molar equivalents;
(j) the volume ratio of antisolvent for a free base of a compound of formula XI to aqueous proton acceptor of the mixture of step b) is in range from 1 to 2, preferably 1.2 to 1.8, more preferably 1.3 to 1.6, in particular 1.35 to 1.5;
(k) the proton donator is selected from the group consisting of inorganic or organic acids, preferably inorganic or organic acids having a pKₛ within a range of 3.0 to 6.0, more preferably ammonium salts, formic acid and acetic acid, even more preferably NH₄Cl, (NH₄)₂SO₄ and acetic acid, and in particular acetic acid.

5. The process according to any one of claims 1 to 4, wherein the solvate form of a salt of compound of formula XI provided in step a) is **characterized by** either one or a combination of the following features (x) and (y):
(x) a first solvent and a second solvent are incorporated into said solvate form of a salt of compound of formula XI wherein the first solvent is different from the second solvent, preferably said first solvent is an alcohol and said second solvent is a hydrocarbon or halocarbon, more preferably said first organic solvent is a C₁-C₄-alcohol and said second organic solvent is a C₆-C₁₀-hydrocarbon comprising a benzene moiety or a C₁-C₁₀-halocarbon optionally comprising a benzene moiety, even more preferably said first solvent is methanol or ethanol and said second solvent is toluene, methylene chloride, chloroform or chlorobenzene, and in particular, said first solvent is methanol and said second solvent is methylene chloride.
(y) said solvate form of compound of formula XI comprises said first solvent in an amount of percent by weight which is lower than the amount of percent by weight of the second solvent, preferably said solvate form of compound of formula XI comprises said first solvent in an amount of about 0.1 to 8 percent by weight and said second solvent in an amount of about 3 to 15 percent by weight relative to the salt of compound of formula XI, more preferably said solvate form of compound of formula XI comprises said first solvent in an amount of about 1 to 5 percent by weight and said second solvent in an amount of about 7.7 to 10 percent by weight relative to the salt of compound of formula XI

6. A hydrate of a compound of the formula XI'

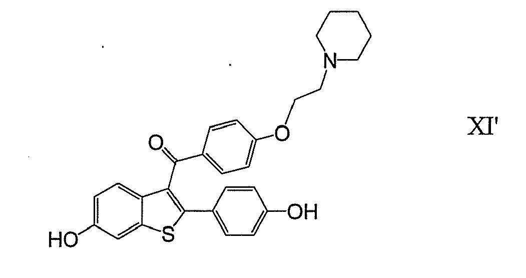

7. The hydrate according to claim 6, **characterized by** either one or a combination of the following features (i) and (ii):
(i) said hydrate comprises 2.8 to 4.7 % by weight of water relative to a hydrate of a compound of formula XI, preferably 3.2 to 4.2 % by weight, more preferably 3.4 to 3.9 % by weight;
(ii) said hydrate is free of antisolvent for a free base of a compound of formula XI' or preferably comprises said antisolvent in an amount of up to 15 % by weight relative to a hydrate of a compound of formula XI, more preferably up to 9.2 % by weight, even more preferably up to 7.4 % by weight and in particular 0.1 to 4.9 % by weight; preferably, said antisolvent is acetone.

8. The hydrate according to claim 6 or 7, wherein said hydrate exhibits a X-ray diffraction (XRD) pattern having at least signals at the following 2θ values (±0.5° respectively):
| | | | | | | | | | | |
|---|---|---|---|---|---|---|---|---|---|---|
| **2theta[°]** | 8.13 | 13.13 | 15.18 | 18.11 | 18.39 | 18.65 | 23.07 | 23.49 | 23.76 | 26.02 |

9. A crystalline solvate form of a compound of formula XI' 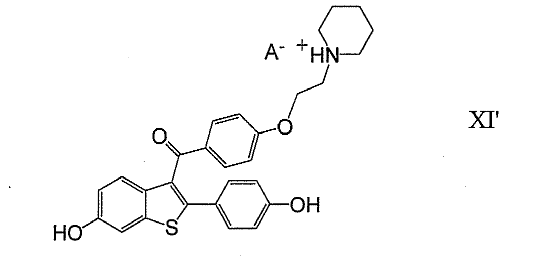 wherein A⁻ is the deprotonated form of an organic or inorganic acid, said crystalline solvate form comprising toluene as a solvent.

10. The crystalline solvate form according to claim 9, **characterized by** either one or a combination of the following features (a) and (b):
(a) said crystalline solvate form comprises toluene in an amount of about 0.1 to 15 percent by weight relative to the solvate form of a salt of compound of formula XI', preferably 3 to 12 percent by weight, more preferably 5 to 10 percent by weight and in particular 7 to 8.5 percent by weight;
(b) A⁻ is selected from the group consisting of oxalate, succinate, lactate, malonate, Cl⁻, Br⁻ and SO₄²⁻, preferably Cl⁻, Br⁻ and SO₄²⁻, more preferably Cl⁻, Br⁻, and even more preferably Br⁻.

11. The crystalline solvate form according to claim 9 or 10, wherein said crystalline solvate form wherein A⁻ is Br⁻ exhibits a X-ray diffraction (XRD) pattern has at least signals at the following 2θ values (±0.5° respectively)⁻:
| | | | | | | | | | | |
|---|---|---|---|---|---|---|---|---|---|---|
| **2theta[°]** | 13.34 | 16.29 | 17.55 | 18.2 | 18.46 | 20.5 | 20.79 | 23.32 | 23.53 | 25.5 |

12. Use of a hydrate of a compound of formula XI 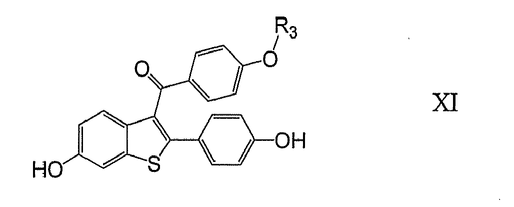 wherein R₃ is 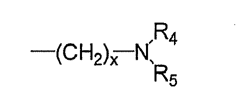 wherein x = 1 to 20, and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring,
in a process for preparing a pharmaceutically active agent.

13. A process for preparing a non-solvated pharmaceutically acceptable salt of a compound of formula XI 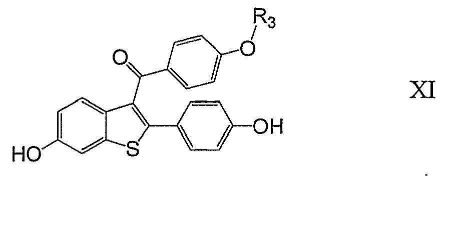 wherein R₃ is 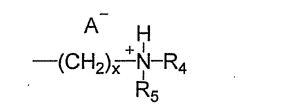 , wherein x = 1 to 20, and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring,
wherein A⁻ is the deprotonated form of an organic or inorganic acid,
at least comprising the steps of:
i) providing a hydrate of a compound of formula XI 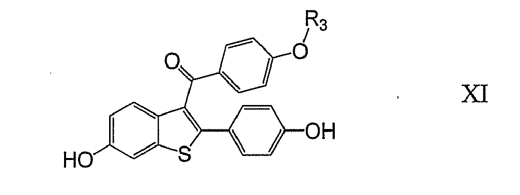 wherein R₃ is 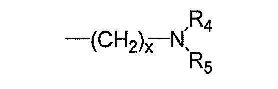 , wherein x = 1 to 20, and R₄ and R₅ independently from each other represent substituted or unsubstituted alkyl, substituted or unsubstituted aryl, substituted or unsubstituted alkylaryl, or R₄ and R₅ cooperatively with the N-atom form a part of a 5- to 10-membered ring,
ii) dissolving said hydrate of step i) in organic solvent,
iii) adding a proton donor in order to quantitatively convert said hydrate of compound of formula XI into a pharmaceutically acceptable salt of a compound of formula XI,
iv) optionally seeding the mixture resulting from step v) in order to induce crystallisation,
v) allowing the pharmaceutically acceptable salt of compound of formula XI to precipitate or to crystallize, optionally under agitation, and
vi) separating the pharmaceutically acceptable salt of a compound of formula XI.

14. The process according to claim 13, wherein said hydrate of a compound of formula XI provided in step i) is **characterized by** either one or a combination of the following structural features (a) to (c):
(a) said hydrate comprises 2.8 to 4.7 % by weight of water relative to hydrate of a compound of formula XI, preferably 3.2 to 4.2 % by weight, more preferably 3.4 to 3.9 % by weight;
(b) said hydrate comprises an antisolvent for a free base of a compound of formula XI' in an amount of up to 15 % by weight relative to a hydrate of a compound of formula XI, preferably up to 9.2 % by weight, more preferably up to 7.4 % by weight and in particular 0.1 to 4.9 % by weight; preferably, said antisolvent is acetone;
(c) said hydrate is a hydrate as defined in any one of claims 6 to 8.

15. The process according to claim 13 or 14, **characterized by** either one or a combination of the following features (d) to (f):
(d) wherein in step ii), said organic solvent forms an azeotrope with the water and/or the optional antisolvent incorporated in said hydrate form, preferably said azeotrope in step ii) is formed under normal or reduced pressure, more preferably under reduced pressure;
(e) in step ii), the resulting mixture is heated prior to step iii), preferably heating is under reflux conditions;
(f) the water and the optional antisolvent incorporated into said hydrate of a compound of formula XI is/are removed by azeotropic distillation of the mixture resulting from step ii), preferably said azeotropic distillation is carried out at reduced pressure.

16. The process according to any one of claims 13 to 15, wherein procedural step ii) is **characterized by** either one or a combination of the following features (x) and (y):
(x) said organic solvent is an alcohol, preferably a water-miscible alcohol, more preferably a C₁-C₄-alcohol, even more preferably methanol or ethanol, and in particular methanol;
(y) said organic solvent of step ii) is free of water or preferably comprises a predetermined amount of water, more preferably said amount of water is predetermined by a volume ratio of water and organic solvent of up to 0.5, even more preferably 0.24 to 0.44, yet even more preferably 0.27 to 0.40, and in particular 0.29 to 0.38.

17. The process according to any one of claims 13 to 16, wherein said proton donor used in step iii) is selected from the group consisting of oxalic acid, succinic acid, lactic acid, malonic acid, HCl, HBr and H₂SO₄, more preferably HCl, HBr and H₂SO₄, even more preferably HCl and HBr, and in particular HBr.
